# EUROPEAN PATENT SPECIFICATION

(11) **EP 2 884 967 B1**
(45) Date of publication and mention of the grant of the patent: **01.02.2017**
(21) Application number: 13752800.6
(22) Date of filing: 16.08.2013
(51) Int. Cl.: A61K 9/16, A61K 9/20, A61K 9/48, A61K 9/50, A61K 31/13

(54) **PHARMACEUTICAL COMPOSITIONS OF MEMANTINE**
PHARMAZEUTISCHE ZUSAMMENSETZUNGEN AUS MEMANTIN
COMPOSITIONS PHARMACEUTIQUES DE MÉMANTINE

(30) Priority: 16.08.2012 US 201261683875 P; 10.06.2013 US 201361833348 P
(43) Date of publication of application: 24.06.2015
(73) Proprietor: Teva Pharmaceutical Industries Ltd., 49131 Petah Tiqva (IL)
(72) Inventor: KAGAN, Elena, Rehovot (IL); SHAHAR, Nitzan, 45805 Kibbutz Tel-Yitzhak (IL); HARONSKY, Elina, Rosh Ha'ain (IL); DEROSA, Gregg, R., Souderton, PA 18964 (US)
(74) Representative: D Young & Co LLP
(86) International application number: PCT/US2013/055420
(87) International publication number: WO 2014/028878

(56) References cited:
- EP-A1- 2 517 696
- WO-A1-2011/038574
- WO-A2-2012/101653
- US-A1- 2006 142 398

## Description

### Field of the Invention

The present invention relates to oral dosage forms comprising Memantine or a pharmaceutically acceptable salt thereof, pharmaceutical formulations comprising the oral dosage forms, and methods for treating comprising the oral dosage forms and formulations.

### Background of the Invention

Memantine is reportedly an orally active NMDA receptor antagonist. The reported IUPAC name for memantine hydrochloride is 3,5-dimethyladamantan-1-amine hydrochloride. Memantine HCl has the structure,

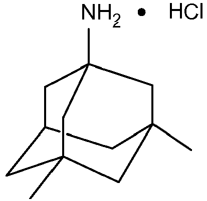

Memantine HCl is currently marketed by Forest in the form of film coated tablets under the trade name NAMENDA®. In Europe, memantine hydrochloride is marketed by Merz (AXURA®) and Lundbeck (EBIXA®). Memantine 10 mg twice daily is the FDA-approved regimen for the treatment of moderate to severe Alzheimer's disease.

NAMENDA XR™ (memantine hydrochloride) was approved by the U.S. Food and Drug Administration for the treatment of moderate to severe dementia of the Alzheimer's type. NAMENDA XR is a 28 mg once-daily extended-release formulation of NAMENDA.

Immediate release pharmaceutical formulations comprising memantine are described in various publications including, inter alia, US 2006/198884 and US 2010/028427.

Modified release pharmaceutical formulations comprising memantine are described in various publications including, inter alia, US 5,382,601, US 6,194,000, US 7,619,007, US 8,039,009 US 8,168,209, US 2007/065512 and US 2010/266684.

Alzheimer's disease is a progressive, degenerative brain disease. As the disease progresses, patients and caregivers face increasing problems with medication adherence. Given Alzheimer's relentlessly progressive nature, newer and more effective therapies for Alzheimer's disease are needed.

### Summary of the Invention

The present invention provides modified release solid oral dosage forms comprising a distinguishably high amount of memantine or a pharmaceutically acceptable salt thereof while avoiding undesirable side effects, particularly CNS side effects. The high doses in the solid oral dosage forms of the present invention are adapted to achieve therapeutically effective steady-state concentrations at a greater interval between dosing than presently employed, while maintaining safety requirements and improving patient compliance.

The present invention provides modified release solid oral dosage forms comprising a therapeutically effective amount of memantine or a pharmaceutically acceptable salt thereof, and at least one pharmaceutically acceptable rate controlling excipient.

In particular, the present invention provides a modified release solid oral dosage form comprising a therapeutically effective amount of memantine or a pharmaceutically acceptable salt thereof, and at least one pharmaceutically acceptable rate controlling excipient, wherein the solid oral dosage form is adapted for administering with an interval between doses of 5 days or above 5 days to a patient in a need thereof, and wherein the solid oral dosage form:
(a) provides *an in vivo* plasma profile at steady state comprising a Cₘₐₓ of about 160 ng/ml or less, a Cₘᵢₙ of more than about 30 ng/ml, and an AUCₜₐᵤ of more than about 14,000 ng h/ml, and/or
(b) has a dissolution profile of: not more than 45% at 24 hours, not more than 70% at 48 hours, and not more than 80% at 55 hours.

The modified release solid oral dosage forms of the present invention provide an *in vivo* plasma profile at steady state comprising Cₘₐₓ of about 160 ng/ml or less, Cₘᵢₙ of more than about 30 ng/ml, AUCₜₐᵤ of more than about 14,000 ng h/ml, and Tₘₐₓ of at least about 36 hours.

The amount of memantine or a pharmaceutically acceptable salt thereof in the modified release solid oral dosage forms of the present invention is preferably up to 200 mg. Preferably, the amount is at least 112 mg, at least 140 mg, at least 160 mg, at least 170 mg, at least 180 mg, or at least about 190 mg of memantine or a pharmaceutically acceptable salt thereof. More preferably, the amount is from about 112 mg to about 200 mg, from about 140 mg to about 200 mg, from about 160, 170, 180 or 190 mg to about 200 mg. In another preferred embodiment, the amount is from about 160 mg to about 190 mg or from about 170 mg to about 190 mg.

The present invention further provides modified release solid oral dosage forms comprising at least about 112 mg, e.g. about 140 mg of memantine or a pharmaceutically acceptable salt thereof, and at least one pharmaceutically acceptable rate controlling excipient, wherein the solid oral dosage form is adapted for administering once weekly to a patient in a need thereof, and wherein the solid oral dosage form provides *an in vivo* plasma profile at steady state comprising a Cₘₐₓ of about 160 ng/ml or less.

In a preferred dosage form of the invention, the memantine or pharmaceutically acceptable salt thereof is present in both immediate release form and extended release form, and may be in the form of coated beads. According to this aspect, of the invention, the dosage form comprises: (i) an immediate release component comprising immediate release memantine or a pharmaceutically acceptable salt thereof, and (ii) an extended release component comprising extended release memantine or a pharmaceutically acceptable salt thereof. Preferably, the immediate release component (i) is in the form of beads comprising immediate release memantine or a pharmaceutically acceptable salt thereof, and the extended release component (ii) is in the form beads comprising extended release memantine or a pharmaceutically acceptable salt thereof, wherein beads comprise an extended-release coating containing at least one rate controlling excipient, i.e. the formulation contains two populations (IR and ER) of memantine or a pharmaceutically acceptable salt thereof. These dosage forms may be in the form of capsules or tablets.

Alternatively, another preferred dosage form of the present invention comprises memantine or pharmaceutically acceptable salt thereof, wherein the memantine is provided in a matrix comprising a mucoadhesive agent. In this embodiment, the mucoadhesive agent functions as the rate controlling excipient as well as enabling the dosage form to be retained in the body for an extended period of time. Preferably the dosage forms are monolithic, i.e. do not contain layers, and more preferably, the dosage form is a tablet.

The present invention provides methods for treating mild, moderate or severe Alzheimer's dementia, or neuropathic pain, wherein the method comprises administering a modified release solid oral dosage form or pharmaceutical formulation according to the present invention to a patient in need thereof.

The present invention provides modified release solid oral dosage forms or pharmaceutical formulations according to the present invention for use in the treatment of mild, moderate or severe Alzheimer's dementia, or neuropathic pain.

The present invention provides modified release solid oral dosage forms or pharmaceutical formulations according to the present invention for use in the manufacture of a medicament for treating mild, moderate or severe Alzheimer's dementia, or neuropathic pain.

### Brief Description of the Drawings

Figure 1 shows the dissolution profile of a 168 mg dose with 5%IR/95%ER (with 30% polymer) population administered once weekly (QW).
Figure 2 shows the concentration over time curve following administration of 140 mg of memantine QW with a zero order release rate. The P5 and P95 refer the 5% and 95% confidence limits for a 28 mg QD dose.
Figure 3 shows the concentration over time curve following administration of 168 mg of memantine QW with 10IR/90(ER 30%) population compared to 28 mg QD. The P5 and P95 refer the 5% and 95% confidence limits for a 28 mg QD dose.
Figure 4 shows the concentration over time curve following administration of 168 mg of memantine QW with 5IR/95(ER 30%) population compared to 28 mg QD. The P5 and P95 refer the 5% and 95% confidence limits for a 28 mg QD dose.
Figure 5 shows the concentration over time curve following administration of 196 mg of memantine QW with 10IR/90(ER 30%) population compared to 28 mg QD. The P5 and P95 refer the 5% and 95% confidence limits for a 28 mg QD dose.
Figure 6 shows the concentration over time curve following administration of 196 mg of memantine QW with 5IR/95(ER 30%) population compared to 28 mg QD. The P5 and P95 refer the 5% and 95% confidence limits for a 28 mg QD dose.

### Detailed Description of the Invention

Unless stated otherwise, the following terms and phrases as used herein are intended to have the following meanings.

As used herein, the term "C" refers to the plasma/serum/blood concentration of an active pharmaceutical ingredient, or drug, such as memantine or a pharmaceutically acceptable salt thereof, in a biological sample, such as a patient sample (e.g., blood, plasma, serum, and cerebrospinal fluid). The concentration of the drug in the biological sample may be determined by any standard assay method known in the art. The term C includes such concentrations measurements as the Cₘᵢₙ, Cₘₐₓ, AUC, and Cₛₛ (steady state concentration). Typically the term C refers to the plasma, serum or blood concentration.

As used herein, the term "Cₘₐₓ" refers to the maximum plasma, serum or blood concentration of a drug, such as memantine or a pharmaceutically acceptable salt thereof, in a biological sample reached over one dosing interval at steady state. Accordingly, Cₘₐₓ is the maximum concentration at steady state, C_{max-SS}.

As used herein, the term "Cₘᵢₙ" refers to the minimum plasma, serum or blood concentration of a drug, such as memantine or a pharmaceutically acceptable salt thereof, in a biological sample reached over one dosing interval at steady state.

As used herein, the term "Tₘₐₓ" refers to the time required to reach the maximal plasma, serum or blood concentration ("Cₘₐₓ") of the drug, such as memantine or a pharmaceutically acceptable salt thereof, in a particular patient sample type.

As used herein, the term "AUC" refers to the area under the plasma, serum or blood concentration versus time curve.

As used herein, the term "AUCₜₐᵤ" refers to the area under the curve for a plasma, serum or blood concentration versus time curve of a drug, such as memantine or a pharmaceutically acceptable salt thereof, reached by a given dose over one dosing interval at steady state. The area under the curve is measured for a time tau at steady state, where tau is the length of the dosing interval. The term AUCₜₐᵤ measures the total exposure at steady state for the dosing interval.

As used herein, the term "immediate release" or "IR" means that the escape or release of a drug, such as memantine or a pharmaceutically acceptable salt thereof, from a dosage form (tablet, capsule, pellet, etc.) occurs immediately or as quickly as possible after administration, usually in a few minutes to hours. The drug is released in a single action and the time of action of the drug is limited.

As used herein, the term "modified release" or "MR" means that the escape or release of a drug, such as memantine or a pharmaceutically acceptable salt thereof, from the dosage form (tablet, capsule, pellet, etc.) has been modified so that the release rate is slower than that in an unmodified or immediate release dosage form. Drug release takes place some time after administration and/or for a prolonged period after administration or to a specific target in the body. Drug release may occur over several hours or over several days in order to maintain a therapeutically effective plasma concentration of the drug. Modified release encompasses delayed release (release at a time other than immediately after administration), extended release (release over a prolonged time period), sustained release (rate of drug release is sustained over a period of time), and controlled release (rate of drug release is controlled to get a particular drug concentration in the body) or a combination thereof.

As used herein, the term "rate controlling excipient" refers to an excipient or a combination of excipients present in such amounts sufficient to control the release rate of the drug in the dosage form, for example to reduce the dose-dependent toxicity of a drug, such as memantine or a pharmaceutically acceptable salt thereof. A rate controlling excipient or a combination thereof controls the rate of drug release from a dosage form.

As used herein, the term "at least one pharmaceutically acceptable rate controlling excipient" refers to the presence of one, two, three, four, or more rate controlling excipients in the dosage form. Preferably, one or two rate controlling excipients are employed.

As used herein, the term "memantine" refers to memantine free base. In certain embodiments, memantine also includes any pharmaceutically acceptable salt, such as the HCl salt. Preferably, in any embodiments of the invention as described herein, the memantine is in the form of its hydrochloride salt. More preferably, in any embodiment of the invention as described herein, reference to the amounts and dosage ranges of memantine in the solid oral dosage forms are to the amounts and dosage ranges of memantine hydrochloride.

As used herein, the term "QW" refers to a dosage form suitable for once weekly administration.

As used herein, the term "once weekly" means administering a dose once every seven days. The interval between administrations for once weekly is six days if administration of a dose occurs on the same day each week.

As used herein, the term "QD" refers to a dosage form suitable for once daily administration.

As used herein, the term "Cav" refers to average concentration during a dosing interval. It can be calculated as (AUC(₀₋ₜₐᵤ))/tau.

As used herein, the term "DFL" refers to degree of fluctuation. It can be calculated as 100*(Cₘₐₓ-Cₘᵢₙ)/Cav.

As used herein, reference to a total weight of a dosage form refers to the total weight of a tablet (excluding any non functional coating such as cosmetic coating), and in the case of a capsule, refers to the total weight of the capsule contents, excluding the weight of the capsule itself.

As used herein, unless otherwise indicated, references to percentages refer to weight percent.

Capsule sizes refer to standard sizes known to those skilled in the art. For example, a capsule size of -00- refers to a capsule with a volume of from about 0.9 ml to about 1 ml, typically about 0.95 ml; a capsule size of -0- refers to a capsule with a volume of from about 0.6 ml to about 0.7 ml, typically about 0.68 ml; and a capsule size of -1- refers to a capsule with a volume of from about 0.4 ml to about 0.6 ml, typically about 0.5 ml.

As used herein, the term "bioavailability" refers to the rate and extent to which an active pharmaceutical ingredient is absorbed from a dosage form and becomes available at the site of action.

The present invention provides modified release solid oral dosage forms comprising a therapeutically effective amount of memantine or a pharmaceutically acceptable salt thereof, and at least one pharmaceutically acceptable rate controlling excipient, wherein the solid oral dosage forms are adapted for administering with an interval between doses of 5 days, or more e.g. 6-8 days, preferably 6, 7 or 8 days and more preferably 6 days, to a patient in a need thereof, and wherein the solid oral dosage forms provide *an in vivo* plasma profile at steady state comprising a Cₘₐₓ of about 160 ng/ml or less, a Cₘᵢₙ of more than about 30 ng/ml, and an AUCₜₐᵤ of more than about 14,000 ng h/ml. For example, it will be appreciated that an interval of 6 days between doses would enable the drug to be administered weekly to the patient, on the same day of the week.

The present invention further provides modified release solid oral dosage forms comprising at least about 112 mg, e.g., about 140 mg of memantine or a pharmaceutically acceptable salt thereof, and at least one pharmaceutically acceptable rate controlling excipient, wherein the solid oral dosage forms are adapted for administering once weekly to a patient in a need thereof, and wherein the solid oral dosage form provides *an in vivo* plasma profile at steady state comprising a Cₘₐₓ of about 160 ng/ml or less.

The present invention further provides modified release solid oral dosage forms comprising at least about 112 mg, or at least about 140 mg of memantine or a pharmaceutically acceptable salt thereof, and at least one pharmaceutically acceptable rate controlling excipient, wherein the solid oral dosage form is adapted for administering once weekly, most preferably on the same day every week, to a patient in a need thereof, and wherein the solid oral dosage form provides *an in vivo* plasma profile at steady state comprising a Cₘₐₓ of about 160 ng/ml or less, a Cₘᵢₙ of more than about 30 ng/ml, Tₘₐₓ of at least about 36 hours, and an AUCₜₐᵤ of more than about 14,000 ng h/ml.

In one embodiment, the present invention provides a modified release solid oral dosage form comprising at least about 112 mg, preferably at least about 140 mg, and more preferably at least about 160 mg of memantine or a pharmaceutically acceptable salt of memantine, and at least one pharmaceutically acceptable rate controlling excipient, wherein the solid oral dosage form provides *an in vivo* plasma profile at steady state comprising a Cₘₐₓ of about 160 ng/ml or less.

The amount of memantine or a pharmaceutically acceptable salt thereof in the modified release solid oral dosage forms of the present invention is up to 200 mg. Preferably, the amount is of at least 112 mg, at least 140 mg, at least 160 mg, at least 170 mg, at least 180 mg, or at least about 190 mg of memantine or a pharmaceutically acceptable salt thereof. More preferably, the amount is from about 112 mg to about 200 mg, from about 140 mg to about 200 mg, from about 160, 170, 180 or 190 mg to about 200 mg, for example, 168mg, 176mg, 182mg, 188mg and 196mg.

A pharmacokinetic parameter or combinations of such parameters indicate the bioavailability of an active pharmaceutical ingredient, such as, memantine or a pharmaceutically acceptable salt thereof. Such pharmacokinetic parameters are known to the person skilled in the art. Examples of such parameters include: T_{1/2} (half-life), Cₘᵢₙ, Cₘₐₓ, AUC, AUCₜₐᵤ, Tₘₐₓ, and Cₛₛ (steady state concentration).

In preferred embodiments, the modified release solid oral dosage forms of the present invention comprise the following *in vivo* plasma profile concentrations, at steady state: a Cₘₐₓ of about 145 ng/ml or less or about 135 ng/ml or less, more preferably about 125 ng/ml or less; a Cₘᵢₙ of more than about 30 ng/ml, more preferably greater than about 40 ng/ml, and even more preferably a Cₘᵢₙ of more than about 50 ng/ml.

In a specific embodiment of the present invention, the modified release solid oral dosage forms provide *an in vivo* plasma profile at steady state comprising an AUCₜₐᵤ of more than about 14,000 ng h/ml, preferably more than about 15,000 ng h/ml, most preferably, more than about 16,000 ng h/ml. Optionally, the modified release solid oral dosage forms provide *an in vivo* plasma profile at steady state comprising an AUCₜₐᵤ of more than about 17,000 ng h/ml.

The solid oral dosage forms of the present invention comprise the following *in vivo* plasma profile concentrations at steady state: a Cₘₐₓ from about 100 ng/ml to about 170 ng/ml, preferably from about 100 ng/ml to about 140 ng/ml or from about 100 ng/ml to about 130 ng/ml; a Cₘᵢₙ from about 20 ng/ml to about 125 ng/ml, preferably from about 30 ng/ml to about 125 ng/ml or from about 40 ng/ml to about 125 ng/ml or from about 50 ng/ml to about 125 ng/ml; and an AUCₜₐᵤ from about 10,000 ng h/ml to about 25,000 ng h/ml, preferably from about 12,000 ng h/ml to about 25,000 ng h/ml or from about 14,000 ng h/ml to about 25,000 ng h/ml or from about 15,000 ng h/ml to about 25,000 ng h/ml or from about 16,000 ng h/ml to about 25,000 ng h/ml or from about 17,000 ng h/ml to about 25,000 ng h/ml.

The solid oral dosage forms of the present invention solve the problem of providing a unit dose of memantine for low frequency administration i.e., more than 5 day intervals (preferably 6 day intervals, i.e. preferably once weekly that also provides a therapeutically effective amount of memantine and with minimal side effects, preferably not more than the side effects associated with Namenda XR^{®} (28mg, capsule), most preferably not more than the side effects associated with of Namenda^{®} (10mg, tablet) when these are administered at the recommended dosages (for example once daily for Namenda XR^{®} 28 mg and twice per day for Namenda^{®} 10 mg). Compositions for low frequency administration, for example, once weekly, require higher amounts of memantine. Compositions with higher amounts of memantine would increase undesirable side effects and toxicity because the memantine would be released and transported into the bodily fluids immediately or over a very short period of time after administration. In addition, a therapeutically effective amount of memantine would not be present in the bodily fluids during the prolonged interval between administrations. In order to achieve a desirable combination of pharmacokinetic parameters, higher dose memantine compositions are formulated to control the release of memantine so that a therapeutically effective amount is available in the bodily fluids.

Currently available formulations for memantine, such as those for NAMENDA® and Namenda XR™ which are intended for daily administration, are not suitable for lower frequency administration, for example, for once weekly administration. For example, these formulations do not contain the required amount of memantine or the sufficiently prolonged release characteristics to enable a longer interval between doses. For example, varying the amount of memantine in such formulations in order to provide an acceptable Cₘᵢₙ to be therapeutically effective, would also increase the Cₘₐₓ to a level that is toxic. Lowering the Cₘₐₓ to non-toxic levels in such formulations would result in a Cₘᵢₙ that are too low to be therapeutically effective throughout the interval between administrations. Even if an acceptable Cₘₐₓ and Cₘᵢₙ can be provided, the total exposure (AUC) to memantine during the interval between administration would be too low so that there would be too little memantine in the systemic circulation to achieve appropriate therapeutic activity.

The dosage forms of the present invention solve the problems of providing a desirable combination of pharmacokinetic parameters by modifying the release of memantine by increasing the dose of memantine and including excipient that control the release of memantine. The rate controlling excipients reduce the rate of release of memantine so that the total amount of memantine is not released all at once, but is prolonged over the interval between administrations.

However, the inventors have found that prolonging the release of memantine creates the problem that too little memantine is released early during the interval between administration. As a result, a therapeutically effective amount is not achieved. By including a portion of the total memantine in immediate release form and another portion in extended release form in accordance with the present invention, the inventors have found that it is possible to achieve a sustained release formulation for low frequency administration whilst enabling a therapeutically effective amount of memantine to be achieved soon after administration. The amount of memantine in the immediate release form is a sufficient amount that does not produce a spike in the plasma concentration versus time curve, that is, does not produce a too high Cₘₐₓ that increases toxicity.

The dosage forms of the present invention as described above may further include one or more mucoadhesives to slow the passage of the dosage form through the body e.g. gastrointestinal tract, so that the dosage form remains in the body sufficiently long for all the memantine to be released in the body.

According to a second aspect of the present invention, a sustained release formulation for low frequency administration can also be produced by formulating memantine as a mucoadhesive dosage form, e.g. as a matrix formulation which releases memantine over an extended period while remaining in the gastrointestinal tract. These dosage forms include at least one mucoadhesive excipient providing the required sustained release characteristics to enable low frequency administration as described above. The mucoadhesive dosage forms according to this aspect of the present invention are preferably monolithic, i.e. do not comprise layers.

Treatments of acute and chronic neurological and neuropsychiatric diseases have the problem of treatment compliance because the patient or caretaker may forget to administer the medication. This is especially applicable to the treatment of Alzheimer's disease. The modified release dosage forms of the present invention have the advantage that they can be administered less often and therefore improves compliance.

Accordingly, the oral dosage forms of the present invention provide advantages over other known oral dosages. For example, the oral dosage forms of the present invention can be administered less frequently yet still provide a therapeutic effective amount of memantine and steady state blood levels. The oral dosage forms of the present invention provide reduced pill burden for patients who resist treatment, increase convenience for caregivers who can only check the patient on a weekly basis leading to greater compliance and less burden on family members. In addition, the oral dosage forms of the present invention can be taken with or without food, and for patients who cannot swallow capsules, the oral dosage forms can be sprinkled on applesauce or other food and the entire contents consumed.

The dosage forms described herein are formulated such that the memantine or a pharmaceutically acceptable salt thereof present in the dosage form has an *in vitro* dissolution profile that is slower than that for an immediate release (IR) formulation as well as slower than that for the 28 mg of memantine extended release NAMENDA XR™ (inactive ingredient are sugar spheres, polyvinylpyrrolidone, hypromellose, talc, polyethylene glycol, ethylcellulose, ammonium hydroxide, oleic acid, and medium chain triglycerides in hard gelatin capsules). The dosage forms of the present invention may contain immediate release, sustained or extended release or delayed release components, or combinations thereof. Preferably, the dosage forms of the present invention comprise a combination of an immediate release component and a sustained or extended release component.

The memantine or a pharmaceutically acceptable salt thereof, in the solid oral dosage forms of the first aspect of the present invention can be provided in a modified release form such as controlled or extended release (ER) form, with an immediate release (IR) component. Thus, the solid oral dosage forms of this aspect of the present invention comprise both an IR (immediate release) component and an ER (extended release) component. In a preferred embodiment, the solid oral dosage form of the invention contains at least 90% of the memantine or a pharmaceutically acceptable salt thereof in an extended release form and the remaining memantine or pharmaceutically acceptable salt thereof in an immediate release form. Another specific embodiment of the invention is a modified release solid oral dosage form, wherein at least 95% of the memantine or a pharmaceutically acceptable salt thereof is in an extended release form and the remaining memantine or pharmaceutically acceptable salt thereof is in an immediate release form.

In one preferred embodiment, the solid oral dosage forms of the present invention comprise about 5% to about 20% of the memantine or a pharmaceutically acceptable salt thereof in immediate release form and about 80% to about 95% of the memantine or a pharmaceutically acceptable salt thereof in extended release form. In a more preferred embodiment, the solid oral dosage forms of the present invention comprise about 5% to about 10%, about 5% to about 15% of the memantine or a pharmaceutically acceptable salt thereof in immediate release form and about 85% to about 95%, about 90% to about 95% of the memantine or a pharmaceutically acceptable salt thereof in extended release form.

The immediate release component of the dosage forms of the present invention can comprise a core coated with layer containing memantine or with a pharmaceutically acceptable salt thereof. The IR component can comprise a sugar sphere, which is coated with a layer containing IR memantine or a pharmaceutically acceptable salt thereof. The IR memantine layer can comprise memantine or a pharmaceutically acceptable salt thereof and a binder (preferably hydroxypropyl methyl cellulose, preferably Methocel E-5 PR). Alternatively, instead of a core (e.g. a sugar sphere) coated with a memantine (and optionally a binder) layer, the IR component can be formed as a core comprising IR memantine, for example, memantine or a pharmaceutically acceptable salt thereof, preferably in combination with a filler (preferably microcrystalline cellulose, e.g. Avicel PH101).

The extended release component of the dosage forms of the present invention can be prepared by coating the beads forming the immediate release component, as defined above, with a controlled release layer. Preferably the controlled release layer comprises a rate controlling excipient optionally with a plasticizer and/or a pore former. More preferably, the controlled release layer comprises a rate controlling excipient together with a plasticizer. Particularly preferred controlled release layers include: (1) at least one rate controlling polymer (preferably ethyl cellulose, more preferably ethyl cellulose 7cPS, hydroxypropyl methylcellulose, preferably HPMC 6 cPs, or a combination thereof) and at least one plasticiser (preferably triethyl citrate), and (2) a polyacrylate dispersion (preferably an aqueous dispersion of a neutral copolymer based on ethyl acrylate and methyl methacrylate such as Eudragit NE 30D) and at least one pore former and/or an anti-tacking agent

(preferably talc). The talc in the latter formulation, as well as functioning as a pore former, may also function as an anti-tacking agent for the controlled release layer.

The controlled release excipient is preferably present in an amount of about 10 to about 50 wt% relative to the weight of the ER memantine component in the dosage form, more preferably about 15 to about 45 wt% and most preferably about 18 to about 38 wt% relative to the weight of the ER memantine component in the dosage form.

Preferably the weight ratio of control release excipient(s) to plasticizer in the ER memantine component of the dosage form ranges from about 10:1 to about 3:1, more preferably about 8:1 to about 4:1, and most preferably about 6:1 to about 5:1.

The amount of the controlled release layer components (e.g. controlled release polymer and plasticizer) relative to the total weight of the ER memantine component of the dosage form is preferably from about 15 to about 50 wt%, more preferably from about 20 to about 35 wt% and particularly from about 20 to about 30 wt%.

Preferably, the weight ratio of the controlled release excipient to memantine or pharmaceutically acceptable salt thereof in the ER memantine component of the dosage form is about 1 : 1 to about 1 : 5, more preferably about 1 : 1 to about 1 : 4, and particularly about 1 : 1 to about 1 : 3, especially about 1 : 1.5 to about 1 : 2.5.

Preferably the weight ratio of the controlled release layer components (e.g. controlled release polymer and plasticizer) relative to the remaining components in the ER memantine component of the dosage form is about 1 : 5 to about 1: 1, more preferably about 1 : 4 to about 1 : 2, and particularly about 1 : 3.8 to about 1 : 3.

Solid oral dosage forms of the present invention comprising both an IR and ER component can be made by mixing an IR component with an ER component. For example, dosage forms having 10% IR and 90% ER, or 5% IR and 95% ER (designated 10IR/90ER and 5IR/95ER) can be made by mixing corresponding portions of IR and ER. The mixture can be filled into a capsule, or compressed into a tablet. Where the dosage form is a tablet, the IR and ER components can be mixed together, optionally with further excipients (e.g. lubricant, filler and optionally a mucoadhesive, as defined herein - preferably magnesium stearate, lactose, starch, and polyethylene oxide) before being compressed into tablets.

Modified release dosage forms can be made by, but not limited to, making pellets of different thicknesses so that the thinnest release the drug first and the thickest last, including a slow dissolving matrix or coating, including a non-dissolving coating around a tablet or capsule with small holes to let the drug out (by diffusion or solvation), controlling release of the drug by diffusion through a coating or matrix or by erosion of the matrix or coating by a process dependent on, for example, a particular condition such as the presence of enzymes or a particular pH. Modified release dosage forms have higher amounts of the drug than the amount present in an unmodified or immediate release dosage form.

The dissolution rate of the dosage forms of the present invention are typically of not more than 35% at 24 hours, not more than 70% at 48 hours, or not more than 80% at 55 hours. In a preferred embodiment, the modified release solid oral dosage forms provide a dissolution rate of not more than 70% at 50 hours, or a dissolution rate of more than 70% at 72 hours or a dissolution rate of more than about 80% at about 96 hours. As used herein, dissolution rates are measured using a USP type 1 (basket) dissolution system at 100 rpm, 900ml, 0.2% NaCl in 0.1N HCl pH=1.2, at a temperature of 37±0.5° C.

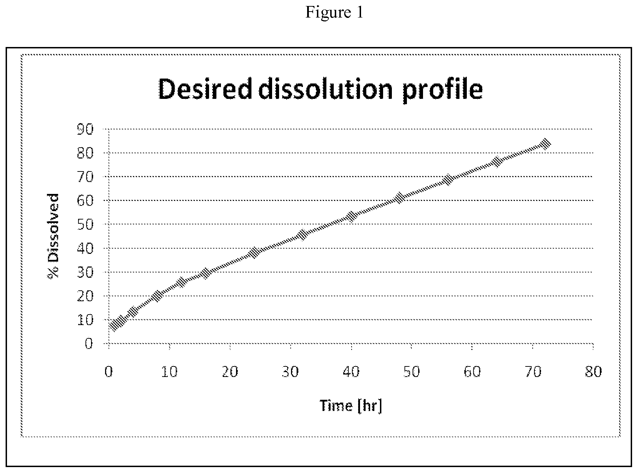

Desirably, a specific dosage form described herein has an *in vitro* profile that is substantially identical to the dissolution profile shown in Figure 1. For example, the modified release solid oral dosage forms provide a dissolution rate of:
(i) about 15% to about 25% after 10 hours, about 35% to about 45% after 24 hours and about 55% to about 65% after 48 hours
(ii) preferably about 30% to about 40% after 20 hours, about 50% to about 60% after 40 hours and about 65% to about 80% after 60 hours,
(iii) more preferably about 10% to about 20% after 5 hours, about 40% to about 50% after 30 hours and about 65% to about 75% after 60 hours.

In preferred embodiments, the modified release solid oral dosage forms of the present invention provide *in vivo* plasma profiles at steady state which are further characterized by a memantine or a pharmaceutically acceptable salt thereof Tₘₐₓ of at least about 36 hours, more preferably a memantine or a pharmaceutically acceptable salt thereof Tₘₐₓ of about 36 to 96 hours. Most preferably, the memantine or a pharmaceutically acceptable salt thereof Tmax is of about 48 to 72 hours.

The modified release solid oral dosage forms of the present invention can be adapted for administration with an interval between doses of above 5 days (preferably, of 6 days) to a patient in a need thereof. Preferably, the dosage form is adapted for administration once every 7 days. In a preferred embodiment, the interval between administrations is 5 days, 6 days or 7 days. For example, administration once every 7 days (i.e. with an interval of 6 days) enables administration of the drug on the same day of the week, thus facilitating patient compliance.

The solid oral dosage forms of the present invention include all pharmaceutically acceptable salts of memantine. Preferably, the memantine is in its hydrochloride salt form or in its sulfate salt form. More preferably the memantine is in the form of memantine hydrochloride.

The modified release solid oral dosage form of the present invention is suitable for administration in a one unit dosage form. Oral dosage forms for the purpose of the present invention include capsules, tablets, pellets, granules, powders and combinations thereof. Preferably, oral dosage forms of the present invention are in the form of capsules, tablets, pellets or granules. Particularly preferred are oral dosage forms in the form of capsules or tablets. Optionally, if the dosage form is a capsule, the memantine or a pharmaceutically acceptable salt thereof is provided in the form of coated beads. Typically, if the modified release solid oral dosage form is in a capsule form, the dosage form comprises from about 15% to about 25% by weight of memantine or a pharmaceutically acceptable salt of memantine. Capsule size of the present invention is preferably smaller than -00-, more preferably, -0- or smaller, most preferably -1- or smaller.

Typically, if the modified release solid oral dosage form is in a tablet form, the dosage form comprises from about 10% to about 20% by weight (preferably about 10% to about 18 wt%), or about 15% to about 35% by weight of memantine or a pharmaceutically acceptable salt of memantine. In other embodiments, the modified release solid oral dosage form comprises memantine or the pharmaceutically acceptable salt thereof from about 15% to about 25% by weight of the total dosage form. Typically for dosage forms of the present invention in capsule form, the dosage form comprises from about 20% to about 60%, preferably from about 25% to about 50%, and more preferably from about 28% to about 50%, by weight of memantine or a pharmaceutically acceptable salt of memantine.

The pharmaceutically acceptable rate controlling excipient of the modified release solid oral dosage form according to the first aspect of the present invention can be a polymeric material or combinations of one or more polymeric materials. Preferably, the polymeric material is selected from a group consisting of: polyethylene oxide, ethyl cellulose (e.g., preferably ethylcellulose having a viscosity of about 4 to about 10 cPs, particularly about 7 cPs), hydroxypropyl methylcellulose (HPMC, preferably having a viscosity of about 4 to about 9 cPs, more preferably about 5 to about 8 cPS, and most preferably about 5 to about 7 cPs and particularly about 6 cPs), polyvinyl alcohol (PVA, preferably polyvinyl alcohol 205, 523, 540, 203S, 205S, 523S and 540S), polyvinylpyrrolidone (PVP, preferably Povidone K 12, Povidone K 17, Povidone K 25, Povidone K 30 and Povidone K 90, more preferably Povidone K 25, Povidone K 30 and Povidone K 90). These polymeric materials are preferably combined with a plasticizer to form the controlled (extended) release layer. Suitable plasticizers include those selected from the group consisting of: polyethylene glycol, triethyl citrate, tributyl citrate, glycerine, dibutyl sebacate, triacetin and diethylphthalate. Other suitable rate controlling excipients include polyacrylates, polymethacrylates, ethyl acrylate-methyl methacrylate copolymers (such as Eudragit RS or NE), hydroxypropyl cellulose (HPC, preferably having a viscosity of about 4 to about 9 cPs, more preferably about 5 to about 8 cPS, and most preferably about 5 to about 7 cPs) and a mixture thereof. A particularly preferred rate controlling excipient is a copolymer of ethyl acrylate and methyl methacrylate, for example an aqueous dispersion of a neutral copolymer based on ethyl acrylate and methyl methacrylate (e.g. Eudragit, preferably Eudragit NE30D, which is a 30% aqueous dispersion of the above).

Preferably, the pharmaceutically acceptable rate controlling excipient is selected from a group consisting of: polyethylene oxide, ethyl cellulose (e.g. ethylcellulose having a viscosity of about 4 to about 10 cPs), hydroxypropyl methylcellulose (HPMC), ethyl acrylate-methyl methacrylate copolymers (Eudragit^{®}), and a mixture thereof. In certain preferred embodiments, the rate controlling excipient is a combination of at least two polymeric materials. For example, in some preferred embodiments, the rate controlling excipient is a combination of ethyl cellulose (preferably having a viscosity of about 4 to about 10 cPs, and more preferably about 6 to about 8 cPs), and hydroxylpropylmethyl cellulose (preferably having a viscosity of about 4 to about 9 cPs, more preferably about 5 to about 8 cPS, and most preferably about 5 to about 7 cPs). In some preferred embodiments, the pharmaceutically acceptable rate controlling excipient is a combination of polyethylene oxide and Eudragit^{®}, a combination of ethyl cellulose (having a viscosity of about 4 to about 10 cPs), hydroxypropyl methylcellulose (having a viscosity of about 4 to about 9 cPs) and triethyl citrate, or a combination of polyethylene oxide.

The total amount of the rate controlling excipient, i.e., the polymeric material and other rate controlling excipient, is about 8% to about 60% of the total weight of the dosage form. In a preferred embodiment, the total amount of the rate controlling excipient is from about 8% to about 50% of the total weight of the dosage form, or from about 8% to about 40% of the total weight of the dosage form, or from about 8% to about 30% of the total weight of the dosage form, or from about 8% to about 20% of the total weight of the dosage form, or from about 50% to about 60% of the total weight of the dosage form. Preferable, total amount of the rate controlling excipient is about 19% to about 40% of the total weight of the dosage form, or from about 19% to about 30% of the total weight of the dosage form, or from about 19% to about 25% of the total weight of the dosage form, or from about 30% to about 60% of the total weight of the dosage form, or from about 30% to about 50% of the total weight of the dosage form, or from about 30% to about 40% of the total weight of the dosage form, or from about 40% to about 60% of the total weight of the dosage form, or from about 50% to about 60% of the total weight of the dosage form. Preferably, in the ER memantine component, the memantine or pharmaceutically acceptable salt thereof is present in an amount of about 8 to about 60 wt%, preferably about 10 to about 50 wt%, relative to the ER component. The amount of the rate controlling polymer is about 60% to about 100%, about 70 to about 90%, about 70% to about 80% of the weight of the ER component of the dosage form.

The amount of the rate controlling polymer in the controlled release layer, i.e. not including extragranular part is about 60% to about 100%, about 70 to about 90%, about 70% to about 80% of the total weight of the controlled release layer.

In the ER component of the dosage form, the amount of the plasticizer is about 10% to about 20%, about 12% to about 16%, about 14% to about 15% of the weight of the controlled release layer (e.g. controlled release polymer and plasticizer).

In a preferred embodiment, the ratio of the amount by weight of memantine or a pharmaceutically acceptable salt thereof to the total amount by weight of the rate controlling excipient in the ER component of the dosage form is from about 1:0.3 to about 1:5.0. Preferably, the ratio is from about 1:0.4 to about 1:0.8, or from about 1:2.5 to about 1:4.0.

Preferably, in the dosage forms of the present invention having an immediate release memantine component and an extended release memantine component, the ER memantine component preferably comprises about 30% to about 40%, preferably about 35% by weight of the other parts of the ER memantine component (e.g. core and drug layer) . Such dosage forms are designated, for example, as 10IR/90 (ER 30%) or 10IR/90 (ER 35%).

The solid oral dosage forms of the first aspect of the present invention can further comprise one or more mucoadhesives as described below.

As indicated above, a second aspect of the invention provides a solid oral dosage form which comprises memantine or a pharmaceutically acceptable salt thereof in an extended release form in which the memantine or pharmaceutically acceptable salt thereof is formulated with at least one mucoadhesive. Thus, in the dosage forms of this aspect of the present invention, the mucoadhesive functions as both the rate controlling excipient and further enables the dosage form to be retained in the body for an extended time. In particular, the memantine or a pharmaceutically acceptable salt thereof is present in a matrix containing a mucoadhesive. The modified release solid oral dosage forms of the present invention can comprise at least one mucoadhesive with or without an immediate release component or an extended release component as described above. For example, the dosage forms of the present invention can comprise at least one mucoadhesive with only an extended release component, or only immediate release component without controlled release layer.

Alternatively according to a second aspect of the present invention, the memantine may be formulated in a matrix with a mucoadhesive. Preferably the dosage form of this aspect of the present invention is in the form of a monolithic tablet.

Mucoadhesives slow the passage of the dosage form through the body so that the dosage form is inside the body during the interval between administrations so that memantine or a pharmaceutically acceptable salt thereof is released in the body. Mucoadhesives are substances that adhere to a biological tissue for an extended period of time by interfacial forces. The biological tissue is a mucous membrane. Mucoadhesion occur when a mucoadhesive contacts and adheres to a membrane by wetting of the mucoadhesive surface or from the swelling of the mucoadhesive. Further adhesion occurs when the mucoadhesive penetrates into the crevice of the membrane surface or when the chains of the mucoadhesive interact with those of the mucus on the membrane. Suitable mucoadhesive are polymers that are water soluble or water insoluble hydrophilic polymers, polymers that have swellable networks, hydrogels, and polymers with groups that can cross-link with other polymers or with a mucous membrane. For example, mucoadhesives can be polyethylene oxide when used in the matrix with the drug.

In the second aspect of the present invention, where the memantine or pharmaceutically acceptable salt thereof is formulated as a matrix with at least one mucoadhesive, the mucoadhesive may be present from about 20% to about 60%, about 30% to about 60%, about 40% to about 60%, and particularly about 50 to about 60 wt%, of the total weight of the dosage form.

The ratio of the amount by weight of memantine or the pharmaceutically acceptable salt thereof to the amount by weight of the mucoadhesive is from about 1:2 to about 1:4, preferably about 1:4.

In preferred embodiments of the formulations of the present invention wherein the dosage form comprises an IR memantine component and an ER memantine component (wherein the ER component contains memantine coated with an ER layer), polyethylene oxide may be included as an extra-granular layer in the ER component, i.e., not in the core or in the designated controlled release layer. When polyethylene oxide is in the extra-granular layer, it functions as a rate controlling excipient, i.e., the release rate is controlled by both the rate controlling excipients in the controlled release layer and in an extra-granular layer.

The modified release solid oral dosage forms of the present invention may further comprise one or more pharmaceutically acceptable carriers or excipients.

Examples of pharmaceutical acceptable excipients are fillers, binders, glidants, lubricants and bases.

Suitable binders include, for example, cellulose polymers (e.g. hydroxypropylmethyl cellulose, hydroxypropylcellulose, methylcellulose and hydroxyethyl cellulose and METHOCEL™), polyvinylpyrrolidone, polyvinyl alcohol, and mixtures thereof. The amount of binder is about 10% to about 20% of the total weight of the dosage form.

Suitable fillers (diluents) include, for example, microcrystalline cellulose (e.g. Avicel), lactose, sorbitol, dextrose, sucrose, mannitol, dibasic calcium phosphate, starch, and mixtures thereof. The amount of the filler is about 15% to about 60% of the total weight of the dosage form, or about 15% to about 50% of the total weight of the dosage form, or about 15% to about 30% of the total weight of the dosage form.

Suitable glidants include, for example colloidal silicon dioxide, magnesium stearate, talc, sodium stearyl fumarate, magnesium carbonate, starch and mixtures thereof. Preferably the glidant is colloidal silicon dioxide. The amount of the glidant is about 0.1% to about 5% of the total weight of the dosage form, about 0.1% to about 3% of the total weight of the dosage form or about 0.1% to about 1% of the total weight of the dosage form.

Suitable lubricants include, for example, sodium stearyl fumarate, stearic acid, magnesium stearate, calcium stearate, zinc stearate, talc, glyceryl behenate, and mixtures thereof. Preferably, the lubricant is magnesium stearate, talc, and mixtures thereof. The amount of the lubricant is about 0.5% to about 5% of the total weight of the dosage form, or about 2% to about 4% of the total weight of the dosage form.

Suitable bases include sodium carbonate.

Tablets in accordance with this invention can be prepared by conventional mixing, comminution, and tabletting techniques that are well known in the pharmaceutical formulations industry. The modified release tablet, for example, may be obtained by direct compression by punches and dies fitted to a rotary tabletting press, ejection or compression molding, granulation followed by compression, or forming a paste and extruding the paste into a mold or cutting the extrudate into short lengths. Preferably, the process used for preparing tablets is direct compression of the blend.

Compression can be accomplished using conventional equipment. Typically, the blend of active ingredients and excipients is passed through a roller apparatus for compaction. However, other means for compacting the API mixture, e.g., compaction into slugs (or "slugging"), may be used.

To achieve the desired modified release rates, the modified release dosage form may be formulated as a polymeric coating or matrix.

In other embodiments, the present invention provides an oral dosage form comprising a plurality of beads, each bead comprising a core comprising an active ingredient and, in the case of the ER memantine component, a rate controlling excipient layer. The modified release beads in accordance with the present invention may be prepared initially as IR beads, with a core, layer of active ingredient, and a seal (i.e. a cosmetic) coating. Alternatively, the IR beads may be formed of a core containing memantine and optionally a filler. The IR beads may then be coated with a modified release component in the form of a rate controlling excipient dispersion and preferably an additional topcoat of polymer (i.e. a cosmetic coat) for aesthetic, handling or stability purposes. The final dosage form, such as a capsule, may contain a different amount of beads depending on the desired dose of the composition.

The beads or bead mixtures may be used, for example, in suspensions, filled into capsules or compressed into tablets. One or more types of modified release beads can be mixed together and encapsulated.

In one embodiment of the invention, the beads are formulated into capsules with the use of an encapsulation machine.

Alternatively, if the dosage form is to be a tablet, the IR and ER beads may optionally be mixed with further excipients (e.g. lubricant, filler and a mucoadhesive, as defined herein - preferably magnesium stearate, lactose, starch, and polyethylene oxide)

The present invention further provides a method for treating a disorder selected from the group consisting of mild, moderate and severe Alzheimer's dementia, and neuropathic pain, wherein the method comprises administering a therapeutically effective amount of a modified release solid oral dosage form of the invention.

The present invention provides modified release solid oral dosage form or pharmaceutical formulation according to the present invention for use in the treatment of mild, moderate or severe Alzheimer's dementia, or neuropathic pain.

The present invention provides modified release solid oral dosage forms or pharmaceutical formulations according to the present invention for use in the manufacture of a medicament for treating mild, moderate or severe Alzheimer's dementia, or neuropathic pain.

The present invention is illustrated by the following examples, which are not intended to limit the scope of the invention. It will be appreciated that various modifications are within the spirit and scope of the invention.

### Examples

The pharmacokinetic model used in the examples below can be represented as follows.

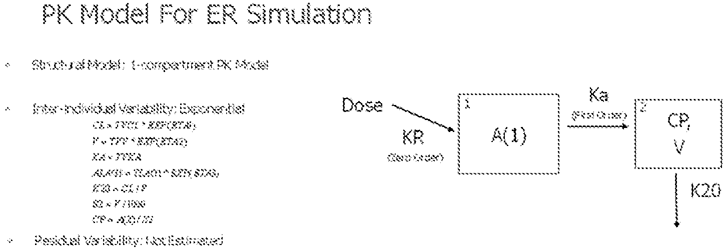

| Parameter | Value | | Parameter | Value |
|---|---|---|---|---|
| Ka | 0,2 1/hr | | | |
| CL | 9,6 L/hr | | ω²_{CL} | 0.0625 |
| V | 828 L | | ω²_{V} | 0.0625 |
| ALAG1 | 1,5 hr | | ω²_{ALG1} | 0.0625 |

### Example 1: Exposure Following Administration Of 140 Mg Of Memantine Once Weekly.

Plasma concentration over time was obtained by convoluting actual (*in vitro*) dissolution data with observed plasma concentration versus time data. The obtained Plasma concentration over time was simulated using a Monte Carlo simulation with a 1-compartment model in NONMEM v 7.1 for 1000 subjects. Phoenix WinNonLin (6.1) was used to perform non-compartmental analysis on the simulated plasma concentration over time. Pharmacokinetic parameters were calculated from the simulated data and are listed below for exposures for once weekly administration of 140 mg of memantine for 7 doses.

| **Dose and Regimen** | **Zero order release rate** | **Mean** | | | | |
|---|---|---|---|---|---|---|
| | | **AUCtau (hr*ng/mL)** | **Cmax (ng/mL)** | **Cmin (ng/mL)** | **Cav (ng/mL)** | **DFL (%)** |
| 140 mg QW | 2.04 mg/hr | 14801 | 133.5 | 46.8 | 88.1 | 98 |

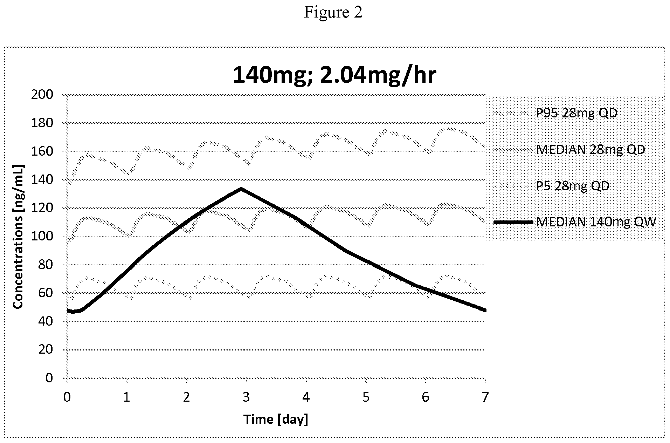

The concentration over time curve following 140 mg of memantine QW administration with a zero order release rate compared to 28 mg QD is shown in Figure 2.

### Example 2: Exposure Following Administration Of Less Than 140 Mg Of Memantine Once Weekly.

Following the procedure in Example 1, pharmacokinetic parameters were calculated for once weekly administration of 28 mg, 56 mg, 84 mg and 112 mg of memantine. The parameters are listed below for exposures to 7 doses.

| **Dose and Regimen** | **Zero order release rate** | **Mean** | | | | |
|---|---|---|---|---|---|---|
| | | **AUCtau (hr*ng/mL)** | **Cmax (ng/mL)** | **Cmin (ng/mL)** | **Cav (ng/mL)** | **DFL (%)** |
| 28 mg QW | 2.04 mg/hr | 2976 | 32.7 | 6.8 | 17.7 | 146 |
| 56 mg QW | 2.04 mg/hr | 5971 | 64.3 | 14.7 | 35.5 | 139 |
| 84 mg QW | 2.04 mg/hr | 8954 | 90.7 | 23.9 | 53.3 | 125 |
| 112 mg QW | 2.04 mg/hr | 11932 | 114.5 | 34.6 | 71.0 | 113 |

### Example 3: Exposure Following Administration Of More Than 140 Mg Of Memantine Once Weekly.

Following the procedure in Example 1, pharmacokinetic parameters were calculated for once weekly administration of 168 mg and 196 mg of memantine. The parameters are listed below for exposures to 7 doses.

| **Dose and Regimen** | **Zero order release rate** | **Mean** | | | | |
|---|---|---|---|---|---|---|
| | | **AUCtau (hr*ng/mL)** | **Cmax (ng/mL)** | **Cmin (ng/mL)** | **Cav (ng/mL)** | **DFL (%)** |
| 168 mg QW | 2.04 mg/hr | 17704 | 146.0 | 60.9 | 105.4 | 81 |
| 196 mg QW | 2.04 mg/hr | 20302 | 160.3 | 75.1 | 120.8 | 71 |

### Example 4: Exposure Following Administration Of 168 Mg Of Memantine Once Weekly.

Plasma concentration over time was obtained by convoluting actual (*in vitro*) dissolution data with observed plasma concentration versus time data. The obtained Plasma concentration over time was simulated using a Monte Carlo simulation with a 2-compartment model in NONMEM v 7.1 for 1000 subjects. Phoenix WinNonLin (6.1) was used to perform non-compartmental analysis on the simulated plasma concentration over time. Pharmacokinetic parameters were calculated from the simulated data and are listed below for exposures for once weekly administration of 168 mg of memantine QW for 11 doses.

| **Dose and Regimen** | **Mean** | | | | |
|---|---|---|---|---|---|
| | **AUCtau (hr*ng/mL)** | **Cmax (ng/mL)** | **Cmin (ng/mL)** | **Cav (ng/mL)** | **DFL (%)** |
| 168mg QW | 14772 | 125.2 | 40.9 | 87.9 | 96 |

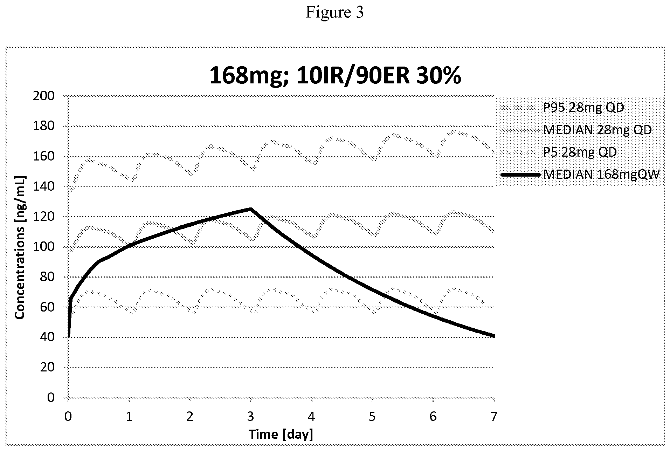

The concentration over time curve following administration of 168 mg of memantine QW with 10 IR / 90 (ER 30%) population compared to 28 mg QD is shown in Figure 3.

### Example 5: Exposure Following Administration Of 168 Mg Of Memantine Once Weekly.

Following the procedure in Example 4, pharmacokinetic parameters were calculated for once weekly administration of 168 mg of memantine with 5IR/95(ER 30%) population. The parameters are listed below for exposures to 11 doses.

| **Dose and Regimen** | **Mean** | | | | |
|---|---|---|---|---|---|
| | **AUCtau (hr*ng/mL)** | **Cmax (ng/mL)** | **Cmin (ng/mL)** | **Cav (ng/mL)** | **DFL (%)** |
| 168mg QW | 14641 | 126.4 | 41.3 | 87.1 | 98 |

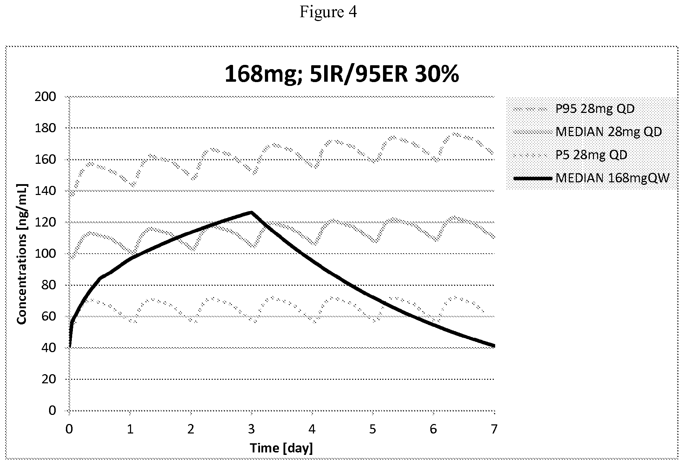

The concentration over time curve following administration of 168 mg of memantine QW with 5IR/95(ER 30%) population compared to 28 mg QD is shown in Figure 4.

### Example 6: Exposure Following Administration Of 196 Mg Of Memantine Once Weekly.

Following the procedure in Example 4, pharmacokinetic parameters were calculated for once weekly administration of 196 mg of memantine with 10IR/90(ER 30%) population. The parameters are listed below for exposures to 11 doses.

| **Dose and Regimen** | **Mean** | | | | |
|---|---|---|---|---|---|
| | **AUCtau (hr*ng/mL)** | **Cmax (ng/mL)** | **Cmin (ng/mL)** | **Cav (ng/mL)** | **DFL (%)** |
| 196mg QW | 17234 | 145.9 | 47.7 | 102.6 | 96 |

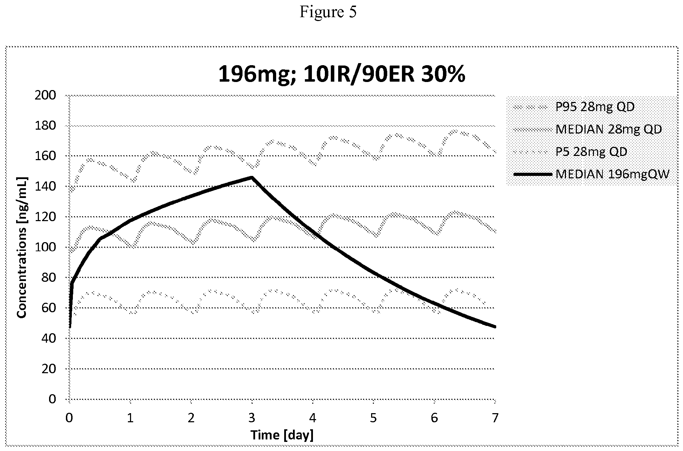

The concentration over time curve following administration of 196 mg of memantine QW with 10IR/90(ER 30%) population compared to 28 mg QD is shown in Figure 5.

### Example 7: Exposure Following Administration Of 196 Mg Of Memantine Once Weekly.

Following the procedure in Example 4, pharmacokinetic parameters were calculated for once weekly administration of 196 mg of memantine with 5IR/95(ER 30%) population. The parameters are listed below for exposures to for 11 doses.

| **Dose and Regimen** | **Mean** | | | | |
|---|---|---|---|---|---|
| | **AUCtau (hr*ng/mL)** | **Cmax (ng/mL)** | **Cmin (ng/mL)** | **Cav (ng/mL)** | **DFL (%)** |
| 196mg QW | 17081 | 147.4 | 48.2 | 101.7 | 98 |

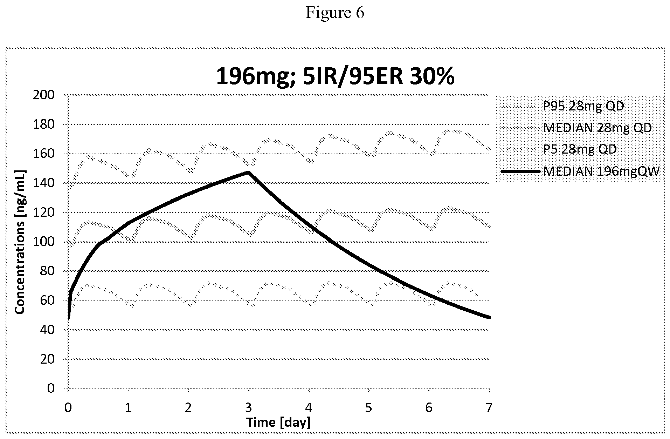

The concentration over time curve following administration of 196 mg of memantine QW with 5IR/95(ER 30%) population compared to 28 mg QD is shown in Figure 6.

### Example 8: Exposure Following Administration Of 176 Mg, 182 Mg And 188 Mg Of Memantine Once Weekly.

Following the procedure in Example 4, pharmacokinetic parameters were calculated for once weekly administration of 176 mg, 182 mg and 188 mg of memantine with 10IR/90(ER 30%) and 5IR/ 5(ER 30%) populations. The parameters are listed below for exposures to 11 doses.

**10IR/90(ER 30%) population**

| **Dose and Regimen** | **Mean** | | | | |
|---|---|---|---|---|---|
| | **AUCtau (hr*ng/mL)** | **Cmax (ng/mL)** | **Cmin (ng/mL)** | **Cav (ng/mL)** | **DFL (%)** |
| 176 mg QW | 15476 | 131.0 | 42.8 | 92.1 | 96 |
| 182 mg QW | 16003 | 135.4 | 44.3 | 95.3 | 96 |
| 188 mg QW | 16531 | 139.9 | 45.8 | 98.4 | 96 |

**5IR/95(ER 30%) population**

| **Dose and Regimen** | **Mean** | | | | |
|---|---|---|---|---|---|
| | **AUCtau (hr*ng/mL)** | **Cmax (ng/mL)** | **Cmin (ng/mL)** | **Cav (ng/mL)** | **DFL (%)** |
| 176 mg QW | 15338 | 132.4 | 43.3 | 91.3 | 98 |
| 182 mg QW | 15861 | 136.9 | 44.8 | 94.4 | 98 |
| 188 mg QW | 16384 | 141.4 | 46.3 | 97.5 | 98 |

The following Examples 9, 10, 11, 13, 14, 15 and 17 illustrate the extended release component of the dosage forms according to the first aspect of the present invention, i.e. wherein the memantine is coated with an extended or controlled release coating. It will be appreciated that the final dosage form can be prepared by mixing the extended release component with an immediate release component, in the appropriate proportions as required, and either encapsulated, e.g. as illustrated in Example 18, or mixed with tableting excipients and compressed, e.g. as illustrated in Example 19. The immediate release components can be made in the same way as the extended release component, but omitting the controlled release layer.

Examples 12 and 16 illustrate dosage forms according to the second aspect of the present invention, i.e. wherein the memantine is provided in a matrix with a mucoadhesive.

### Example 9: Coated Spheres (Extrusion/Spheronized) Filled Into Capsules.

Memantine Hydrochloride and microcrystalline cellulose are mixed into a blend with a high shear mixer. The blend is further granulated with gradual addition of water for a few minutes. The resulting wet mass is extruded through a 0.6-1.0 mm screen, and then spheronized in a spheronizer to create spheroids. The spheroids are dried in a Fluid bed Glatt Dryer till the moisture content is less than about 2%, and optionally sieved to provide particles in a selected size range. The resulting spheroids are coated in a Wurster-equipped Fluidized Bed coater (bottom-spray technology) with Control release layer.

| **Formulation of Beads by Extrusion** | |
|---|---|
| Component | Weight (mg) |
| **Core** | |
| Microcrystalline cellulose (Avicel PH 101) | 210.0 |
| Memantine HCl | 140.0 |
| **Control Release Layer** | |
| Ethylcellulose 7 cPs | 73.5 |
| Hydroxypropylmethyl cellulose 6 cPs | 15.75 |
| Triethyl citrate | 15.75 |
| **Total weight** | 455.0 |

### Example 10: Coated Spheres (DL Coating) Filled Into Capsules.

Sugar spheres are coated with Drug Layer (DL) containing Memantine HCl and HPMC as a binder dissolved in hydro alcoholic solution in a Wurster-equipped Fluidized Bed coater (bottom-spray technology). The second Control Release coating is also performed in Wurster-equipped Fluidized Bed coater.

| **Formulation of Beads by DL Coating** | |
|---|---|
| Component | Weight (mg) |
| **Core** | |
| Sugar spheres | 50.0 |
| **Drug Layer Coating** | |
| Memantine HCl | 140.0 |
| (METHOCEL E-5 PR.(HYPROMEL.USP | 42.0 |
| **Control Release Layer** | |
| Ethylcellulose 7 cPs | 48.72 |
| Hydroxypropylmethyl cellulose 6 cPs | 10.44 |
| Triethyl citrate | 10.44 |
| **Total weight** | 301.6 |

### Example 11: Coated Spheres (DL Coating/Extrusion) Compressed Into Tablets.

Memantine Hydrochloride and microcrystalline cellulose are mixed into a blend with a high shear mixer. The blend is further granulated with gradual addition of water for a few minutes. The resulting wet mass is extruded through a 0.6-1.0 mm screen, and then spheronized in a spheronizer to create spheroids. The spheroids are dried in a Fluid bed Glatt Dryer till the moisture content is less than about 2%, and optionally sieved to provide particles in a selected size range. Alternatively, Memantine HCl pellets can be obtained by Drug Layer (DL) coating as in Example 10. The resulting spheroids are coated in a Wurster-equipped Fluidized Bed coater (bottom-spray technology) with Control release layer. The extended release coated beads are further mixed with a spray-dried compound consisting of 85% alpha-lactose monohydrate and 15% maize starch dry matter (StarlacTM), a non-ionic polyethylene oxide polymer (Polyox WSR- 301) and magnesium stearate and then compressed into tablets.

| **Formulation of Beads compressed into tablets** | |
|---|---|
| Component | Weight (mg) |
| **Core** | |
| Microcrystalline cellulose (Avicel PH 101) | 210.0 |
| Memantine HCl | 140.0 |
| **Control Release Layer** | |
| Polyacrylate dispersion (EUDRAGIT NE 30D) | 86.5 |
| Talc extra fine | 13.5 |
| **Ex**-**granular** | |
| Polyethylene oxide (Polyox WSR-301) | 300.0 |
| STARLAC™ | 300.0 |
| Magnesium stearate | 10.0 |
| **Total weight** | 1060.0 |

### Example 12: Extended Release Matrix Tablets.

Memantine Hydrochloride, non-ionic polyethylene oxide polymer (Polyox WSR-301), microcrystalline cellulose, Lactose, Colloidal silicon dioxide, Sodium Carbonate and Magnesium Stearate are mixed in dry mix and then compressed into tablets. Alternatively, this process can be performed by wet granulation when polyethylene oxide polymer (Polyox WSR- 301) Colloidal silicon dioxide, Sodium Carbonate and Magnesium Stearate are added as ex-granular.

| **Formulation of ER Tablets** | |
|---|---|
| Component | Weight (mg) |
| Memantine HCl | 140.0 |
| Microcrystalline cellulose (Avicel PH 101) | 200.0 |
| Polyethylene oxide (Polyox WSR-301) | 532.5 |
| Lactose | 50.0 |
| Colloidal silicon dioxide | 20.0 |
| Sodium Carbonate | 10.0 |
| Magnesium stearate | 10.0 |
| **Total weight** | 962.5 |

### Example 13: Beads by Extrusion Spheronization Having Higher Strength.

Memantine Hydrochloride and microcrystalline cellulose are mixed into a blend with a high shear mixer. The blend is further granulated with gradual addition of water for a few minutes. The resulting wet mass is extruded through a 0.6-1.0 mm screen, and then spheronized in a spheronizer to create spheroids. The spheroids are dried in a Fluid bed Glatt Dryer till the moisture content is less than about 2%, and optionally sieved to provide particles in a selected size range. The resulting spheroids are coated in a Wurster-equipped Fluidized Bed coater (bottom-spray technology) with Control release layer.

| **Formulation of Beads by Extrusion** | |
|---|---|
| Component | Weight (mg) |
| **Core** | |
| Microcrystalline cellulose (Avicel PH 101) | 294.0 |
| Memantine HCl | 196.0 |
| **Control Release Layer** | |
| Ethylcellulose 7 cPs | 99.2 |
| Hydroxypropylmethyl cellulose 6 cPs | 21.26 |
| Triethyl citrate | 21.26 |
| **Total weight** | 631.7 |

### Example 14: Beads by DL Technology Having Higher Strength.

### For both Formulation A and Formulation B:

Sugar spheres are coated with Drug Layer (DL) containing Memantine HCl and HPMC as a binder dissolved in hydro alcoholic solution in a Wurster-equipped Fluidized Bed coater (bottom-spray technology). The second Control Release coating is also performed in Wurster-equipped Fluidized Bed coater.

| **Formulation of Beads by DL Coating (Formulation A)** | |
|---|---|
| Component | Weight (mg) |
| **Core** | |
| Sugar spheres | 57.5 |
| **Drug Layer Coating** | |
| Memantine HCl | 161.0 |
| (METHOCEL E-5 PR.(HYPROMEL.USP | 57.5 |
| **Control Release Layer** | |
| Ethylcellulose 7 cPs | 56.03 |
| Hydroxypropylmethyl cellulose 6 cPs | 12.00 |
| Triethyl citrate | 12.00 |
| **Total weight** | 346.84 |

| **Formulation of Beads by DL Coating (Formulation B)** | |
|---|---|
| Component | Weight (mg) |
| **Core** | |
| Sugar spheres | 67.85 |
| **Drug Layer Coating** | |
| Memantine HCl | 190.0 |
| (METHOCEL E-5 PR.(HYPROMEL.USP | 67.85 |
| **Control Release Layer** | |
| Ethylcellulose 7 cPs | 66.11 |
| Hydroxypropylmethyl cellulose 6 cPs | 13.80 |
| Triethyl citrate | 13.80 |
| **Total weight** | 409.27 |

### Example 15: Compressed Tablets Formulation.

Memantine Hydrochloride and microcrystalline cellulose are mixed into a blend with a high shear mixer. The blend is further granulated with gradual addition of water for a few minutes. The resulting wet mass is extruded through a 0.6-1.0 mm screen, and then spheronized in a spheronizer to create spheroids. The spheroids are dried in a Fluid bed Glatt Dryer till the moisture content is less than about 2%, and optionally sieved to provide particles in a selected size range. Alternatively, Memantine HCl pellets can be obtained by Drug Layer (DL) coating as in Example 10. The resulting spheroids are coated in a Wurster-equipped Fluidized Bed coater (bottom-spray technology) with Control release layer. The extended release coated beads are further mixed with a spray-dried compound consisting of 85% alpha-lactose monohydrate and 15% maize starch dry matter (StarlacTM), a non-ionic polyethylene oxide polymer (Polyox WSR- 301) and magnesium stearate and then compressed into tablets.

| **Formulation of Beads compressed into tablets** | |
|---|---|
| Component | Weight (mg) |
| **Core** | |
| Microcrystalline cellulose (Avicel PH 101) | 285.6 |
| Memantine HCl | 190.0 |
| **Control Release Layer** | |
| Polyacrylate dispersion (EUDRAGIT NE 30D) | 116.8 |
| Talc extra fine | 18.4 |
| **Ex**-**granular** | |
| Polyethylene oxide (Polyox WSR-301) | 408.0 |
| STARLAC™ | 408.0 |
| Magnesium stearate | 13.6 |
| **Total weight** | 1442.0 |

### Example 16: Extended Release Matrix Tablets.

Memantine Hydrochloride, non-ionic polyethylene oxide polymer (Polyox WSR-301), microcrystalline cellulose, Lactose, Colloidal silicon dioxide, Sodium Carbonate and Magnesium Stearate are mixed in dry mix and then compressed into tablets. Alternatively, this process can be performed by wet granulation when polyethylene oxide polymer (Polyox WSR- 301) Colloidal silicon dioxide, Sodium Carbonate and Magnesium Stearate are added as ex-granular.

| **Formulation of ER Tablets** | |
|---|---|
| Component | Weight (mg) |
| Memantine HCl | 190.0 |
| Microcrystalline cellulose (Avicel PH 101) | 272.0 |
| Polyethylene oxide polymer (Polyox WSR-301) | 728.3 |
| Lactose | 68.0 |
| Colloidal silicon dioxide | 27.0 |
| Sodium Carbonate | 13.6 |
| Magnesium stearate | 13.6 |
| **Total weight** | 1312.5 |

### Example 17:

| **Formulation of Beads by DL Coating** | |
|---|---|
| Component | Weight (mg) |
| **Core** | |
| Sugar spheres | 67.85 |
| **Drug Layer Coating** | |
| Memantine HCl | 190.0 |
| (METHOCEL E-5 PR.(HYPROMEL.USP | 67.85 |
| **Control Release Layer** | |
| Ethylcellulose 7 cPs | 79.8 |
| Hydroxypropylmethyl cellulose 6 cPs | 17.1 |
| Triethyl citrate | 17.1 |
| **Total weight** | 439.7 |

Sugar spheres are coated with Drug Layer (DL) containing Memantine HCl and HPMC as a binder dissolved in hydro alcoholic solution in a Wurster-equipped Fluidized Bed coater (bottom-spray technology). The second Control Release coating is also performed in Wurster-equipped Fluidized Bed coater. The final coated beads are then encapsulated.

### Example 18: Capsule Dosage form containing IR and ER memantine components

| **Formulation of Beads with IR&ER** | |
|---|---|
| Component | Weight (mg) |
| **Core** | |
| Sugar spheres | 67.85 |
| **Drug Layer Coating** | |
| Memantine HCl | 190.0 |
| (METHOCEL E-5 PR.(HYPROMEL.USP | 67.85 |
| **IR Portion** | |
| DL IR Portion 16.30 | |
| **ER Portion** | |
| **Control Release Layer** | |
| Ethylcellulose 7 cPs | 75.8 |
| Hydroxypropylmethyl cellulose 6 cPs | 16.24 |
| Triethyl citrate | 16.24 |
| **Total weight** | 450.28 |

Sugar spheres are coated with Drug Layer (DL) containing Memantine HCl and HPMC as a binder dissolved in hydro alcoholic solution in a Wurster-equipped Fluidized Bed coater (bottom-spray technology). A 95% portion of the IR beads is then coated by additional ER coating. The second Control Release coating is also performed in Wurster-equipped Fluidized Bed coater. Both portions are then mixed and encapsulated.

### Example 19: Tablet dosage form containing IR and ER memantine components

The IR and ER memantine components of Example 18 are mixed with Polyethylene oxide (Polyox WSR-301), STARLAC™ and Magnesium stearate, and compressed into tablets.

### Example 20: Dissolution Procedure.

| | |
|---|---|
| Equipment: | 6-vessel assembly, Apparatus I (Basket) |
| Medium: | 0.2% NaCl in 0.1N HCl |
| Volume: | 900 mL |
| Stirring Rate: | 100 RPM |
| Temperature: | 37°C ± 0.5°C |

## Claims

1. A modified release solid oral dosage form comprising a therapeutically effective amount of memantine or a pharmaceutically acceptable salt thereof, and at least one pharmaceutically acceptable rate controlling excipient, wherein the solid oral dosage form is adapted for administering with an interval between doses of 5 days or above 5 days to a patient in a need thereof, and wherein the solid oral dosage form:
(c) provides an *in vivo* plasma profile at steady state comprising a Cₘₐₓ of about 160 ng/ml or less, a Cₘᵢₙ of more than about 30 ng/ml, and an AUCₜₐᵤ of more than about 14,000 ng h/ml, and/or
(d) has a dissolution profile of: not more than 45% at 24 hours, not more than 70% at 48 hours, and not more than 80% at 55 hours, as measured using a USP type 1 (basket) dissolution system at 100 rpm, 900 ml, 0.2% NaCl in 0.1N HCl pH = 1.2, at a temperature of 37 ± 0.5°C.

2. The modified release solid oral dosage form according to Claim 1 or Claim 2, wherein the dosage form comprises memantine or a pharmaceutically acceptable salt thereof in an amount of at least about 112 mg, at least about 140 mg, at least about 160 mg, at least about 170 mg, or at least about 190 mg.

3. The modified release solid oral dosage form according to Claim 1 or Claim 2, wherein the dosage form comprises memantine or a pharmaceutically acceptable salt thereof in an amount of about 140 to about 190 mg, about 160 to about 190 mg, about 170 mg to about 190 mg, or about 140 to about 200 mg, about 160 to about 200 mg, about 170 to about 200 mg or about 190 to about 200 mg.

4. A modified release solid oral dosage form comprising at least about 112 mg, or at least about 140 mg of memantine or a pharmaceutically acceptable salt of memantine, and at least one pharmaceutically acceptable rate controlling excipient, wherein the solid oral dosage form:
(a) provides an *in vivo* plasma profile at steady state comprising a Cₘₐₓ of about 160 ng/ml or less, and/or
(b) has a dissolution profile of: not more than 45% at 24 hours, not more than 70% at 48 hours, and not more than 80% at 55 hours.

5. A modified release solid oral dosage form according to any of Claims 1-4 adapted for administering once weekly to a patient in a need thereof.

6. A modified release solid oral dosage form according to Claim 5, and wherein the solid oral dosage form:
(a) provides an *in vivo* plasma profile at steady state comprising a Cₘₐₓ of about 160 ng/ml or less, a Cₘᵢₙ of more than about 30 ng/ml, Tₘₐₓ of at least about 36, and an AUCₜₐᵤ of more than about 14,000 ng h/ml, and/or
(b) has a dissolution profile of: not more than 45% at 24 hours, not more than 70% at 48 hours, and not more than 80% at 55 hours.

7. The modified release solid oral dosage form according to any of Claims 4, 5, or 6, wherein the dosage form comprises memantine or a pharmaceutically acceptable salt thereof in an amount of at least about 160 mg, at least about 170 mg, or at least about 190 mg.

8. The modified release solid oral dosage form according to any of Claims 4, 5, 6 or 7, wherein the dosage form comprises memantine or a pharmaceutically acceptable salt thereof in an amount of about 140 to about 190 mg, about 160 to about 190 mg, about 170 mg to about 190 mg, or about 140 to about 200 mg, about 160 to about 200 mg, about 170 to about 200 mg or about 190 to about 200 mg.

9. The modified release solid oral dosage form according to any preceding claim, wherein the dosage form comprises at least about 160 mg of memantine or a pharmaceutically acceptable salt of memantine; or wherein the dosage form comprises at least about 170 mg of memantine or a pharmaceutically acceptable salt thereof; or wherein the dosage form comprises at least about 190 mg of memantine or a pharmaceutically acceptable salt of memantine; or wherein the dosage form comprises up to about 200 mg or memantine or a pharmaceutically acceptable salt of memantine.

10. The modified release solid oral dosage form according to any preceding claim, wherein the solid oral dosage form provides an *in vivo* plasma profile at steady state comprising a Cₘₐₓ of about 100 ng/ml to about 145 ng/ml, about 100 ng/ml to about 135 ng/ml, or about 100 ng/ml to about 125 ng/ml.

11. The modified release solid oral dosage form according to any of Claims 1-9, wherein the solid oral dosage form provides an *in vivo* plasma profile at steady state comprising a Cₘᵢₙ of about 30 ng/ml to about 125 ng/ml, about 40 ng/ml to about 125 ng/ml, or about 50 ng/ml to about 125 ng/ml.

12. The modified release solid oral dosage form according to any of Claims 1-9 wherein the solid oral dosage form provides an *in vivo* plasma profile at steady state comprising a Cₘᵢₙ of more than about 30 ng/ml; or wherein the solid oral dosage form provides an *in vivo* plasma profile at steady state comprising a Cₘᵢₙ of more than about 40 ng/ml; or wherein the solid oral dosage form provides an *in vivo* plasma profile at steady state comprising a Cₘᵢₙ of more than about 50 ng/ml.

13. The modified release solid oral dosage form according to any preceding claim, wherein the solid oral dosage form provides an *in vivo* plasma profile at steady state comprising an AUCₜₐᵤ of more than about 14,000 ng h/ml; or wherein the solid oral dosage form provides an *in vivo* plasma profile at steady state comprising an AUCₜₐᵤ of 15,000 ng h/ml or more, and preferably more than about 15,000 ng h ml; or wherein the solid oral dosage form provides an *in vivo* plasma profile at steady state comprising an AUCₜₐᵤ of more than about 16,000 ng h/ml.

14. The modified release solid oral dosage form according to Claim 13, wherein the solid oral dosage form provides an *in vivo* plasma profile at steady state comprising an AUCₜₐᵤ of about 14,000 ng h/ml to about 25,000 ng h/ml; or wherein the solid oral dosage form provides an *in vivo* plasma profile at steady state comprising an AUCₜₐᵤ of about 15,000 ng h/ml to about 25,000 ng h/ml or about 16,000 ng h/ml to about 25,000 ng h/ml.

15. The modified release solid oral dosage form according to any preceding claim, wherein the solid oral dosage form provides a dissolution rate of not more than 70% at 48 hours, preferably not more than 70% at 72 hours, and more preferably wherein more than about 80%) is achieved after about 96 hours; or wherein the solid oral dosage form provides a dissolution profile of not more than 70% at 50 hours, not more than 75% at 60 hours, and more than about 80% after about 96 hours.

16. The modified release solid oral dosage form according to any preceding claim, wherein the *in vivo* plasma profile at steady state is further **characterized by** a memantine Tₘₐₓ of at least about 36 hours, and preferably a memantine Tₘₐₓ of about 36 to 96 hours; or a Tₘₐₓ of about 48 to 72 hours.

17. The modified release solid oral dosage form according to any preceding claim, wherein the dosage form is adapted for administration with an interval between doses of 5 days or above 5 days, preferably 6 days, to a patient in a need thereof, or for administration once every 7 days to a patient in a need thereof.

18. The modified release solid oral dosage form according to any of Claims 1-17, wherein the pharmaceutically acceptable salt of memantine is hydrochloride salt or sulfate salt, and preferably wherein memantine is in the form of the hydrochloride salt.

19. The modified release solid oral dosage form according to any of Claims 1-18, wherein the dosage form is a one unit dosage form.

20. The modified release solid oral dosage form according to Claim 19, wherein the memantine or pharmaceutically acceptable salt thereof is present in both immediate release form and extended release form.

21. The modified release solid oral dosage form according to Claim 20, wherein the dosage form comprises:
(i) an immediate release component comprising immediate release memantine or a pharmaceutically acceptable salt thereof, and
(ii) an extended release component comprising extended release memantine or a pharmaceutically acceptable salt thereof.

22. The modified release solid oral dosage form according to Claim 20 or Claim 21 wherein: the immediate release component (i) is in the form of beads comprising immediate release memantine or a pharmaceutically acceptable salt thereof, and the extended release component (ii) is in the form beads comprising extended release memantine or a pharmaceutically acceptable salt thereof, wherein beads comprise an extended-release coating containing at least one rate controlling excipient.

23. The modified release solid oral dosage form according to Claim 21 or Claim 22, wherein (i) comprises an inert core, preferably a sugar sphere, coated with memantine or a pharmaceutically acceptable salt thereof, and optionally a binder.

24. The modified release solid oral dosage form according to Claim 23, wherein the binder is selected from the group consisting of: cellulose polymers, hydroxypropylmethyl cellulose, hydroxypropylcellulose, methylcellulose, hydroxyethyl cellulose, polyvinylpyrrolidone, polyvinyl alcohol and mixtures thereof, and preferably wherein the binder is hydroxypropylmethyl cellulose.

25. The modified release solid oral dosage form according to any of Claims 22 to 24, wherein the extended release component (ii) is prepared by coating the immediate release beads of component (i) as defined in any of Claims 40 or 41 with at least one rate controlling excipient.

26. The modified release solid oral dosage form according to any of Claims 1-25, wherein at least 90% by weight of the memantine or a pharmaceutically acceptable salt thereof is in extended release form.; and preferably wherein at least 95% by weight of the memantine or a pharmaceutically acceptable salt thereof is in extended release form.

27. The modified release solid oral dosage form according to any of Claims 1-26, wherein the rate controlling excipient is a polymeric material; preferably wherein the polymeric material is selected from polyethylene oxide, ethyl cellulose (e.g., preferably ethylcellulose having a viscosity of about 4 to about 10 cPs, particularly about 7 cPs), hydroxypropyl methylcellulose (HPMC, preferably having a viscosity of about 4 to about 9 cPs, more preferably about 5 to about 8 cPS, and most preferably about 5 to about 7 cPs and particularly about 6 cPs), polyvinyl alcohol (PVA, preferably polyvinyl alcohol 205, 523, 540, 203S, 205S, 523S and 540S), polyvinylpyrrolidone (PVP, preferably Povidone K 12, Povidone K 17, Povidone K 25, Povidone K 30 and Povidone K 90, more preferably Povidone K 25, Povidone K 30 and Povidone K 90), polyacrylates, polymethacrylates, ethyl acrylate -methyl methacrylate copolymers (preferably Eudragit RS or NE), hydroxypropyl cellulose (HPC, preferably having a viscosity of about 4 to about 9 cPs, more preferably about 5 to about 8 cPS, and most preferably about 5 to about 7 cPs) and a mixture thereof.

28. The modified release solid oral dosage form according to Claim 27,wherein the rate controlling excipient is a combination of two polymeric materials, preferably wherein rate controlling excipient is a combination of ethyl cellulose (preferably having a viscosity of about 5 to about 9 cPs, and more preferably about 6 to about 8 cPs), and hydroxylpropylmethyl cellulose (preferably having a viscosity of about 4 to about 9 cPs, more preferably about 5 to about 8 cPS, and most preferably about 5 to about 7 cPs).

29. The modified release solid oral dosage form according to any of Claims 27 or 28, further comprising a plasticizer; preferably wherein the plasticizer is selected from a group consisting of: polyethylene glycol, triethyl citrate, tributyl citrate, glycerin, dibutyl sebacate, triacetin, diethylphthalate and mixtures thereof, and preferably wherein the plasticizer is triethyl citrate.

30. The modified release solid oral dosage form according to any of Claims 1-27, wherein the rate controlling excipient is an ethyl acrylate-methyl methacrylate copolymer (preferably Eudragit, and more preferably Eudragit NE30D, which is a 30% aqueous dispersion of a copolymer of ethyl acrylate and methyl methacrylate).

31. The modified release solid oral dosage form according to any of Claims 1-30, wherein the total amount of the rate controlling excipients to the total weight of the dosage form is from about 8% to about 60%; from about 8% to about 50%; from about 8% to about 40%; from about 8% to about 30%; from about 8% to about 20%; from about 50% to about 60%>; from about 19%> to about 40%>; from about 19%> to about 30%>; from about 19%) to about 25%; from about 30%> to about 60%>; from about 30%> to about 50%>; from about 30%) to about 40%>; from about 40%> to about 60%>; or from about 50%> to about 60%.

32. The modified release solid oral dosage form according to any of Claims 21-31, wherein the amount of rate controlling excipient is about 10 to about 50 wt%, preferably about 10 to about 45 wt% and more preferably about 15 to about 40 wt% of the extended release component of the dosage form.

33. The modified release solid oral dosage form according to any of Claims 21-32, wherein the amount of memantine or a pharmaceutically acceptable salt of memantine in the extended release component is about 10 to about 60 wt%>, preferably about 10 to about 55 wt% and more preferably about 10 to about 50 wt% relative to the weight of the extended release component.

34. The modified release solid oral dosage form according to any of Claims 21-33, wherein in the extended release component, the weight ratio of the rate controlling excipient(s) to memantine or the pharmaceutically acceptable salt thereof is from about 1 :0.2 to about 1 :5.0, preferably from about 1 :0.3 to about 1 :3.0, and more preferably about 1 :0.3 to about 1 :2.8.

35. The modified release solid oral dosage form according to any of Claims 29-34, wherein in the extended release component, the weight ratio of the plasticizer to rate controlling excipient(s) is from about 1 :2 to about 1 : 10, preferably from about 1 :3 to about 1 :8, and more preferably about 1 :4 to about 1 :7.

36. The modified release solid oral dosage form according to any of Claims 1-35 further comprising a mucoadhesive; preferably wherein the mucoadhesive is selected from the group consisting of water soluble or water insoluble hydrophilic polymers, polymers that have swellable networks, hydrogels, and polymers with groups that can cross-link with other polymers or with a mucous membrane, and preferably wherein the mucoadhesive is polyethylene oxide.

37. The modified release solid oral dosage form according to Claim 36, wherein the mucoadhesive is present in an amount of about 5 to about 60 wt%, about 5 to about 50 wt%, about 5 to about 40wt%, about 5 to about 20 wt%, about 5 to about 15 wt% and most preferably about 5 to about 10 wt%, of the total weight of the dosage form.

38. The modified release solid oral dosage form according to any of Claims 36 or 37, wherein the weight ratio memantine or the pharmaceutically acceptable salt thereof to the mucoadhesive is from about 1 :2 to about 1 :4, preferably about 1 :4.

39. The modified release solid oral dosage form according to any of Claims 1-19 wherein the memantine or pharmaceutically acceptable salt thereof is provided in a matrix comprising a mucoadhesive agent; preferably wherein the mucoadhesive is selected from the group consisting of water soluble or water insoluble hydrophilic polymers, polymers that have swellable networks, hydrogels, and polymers with groups that can cross-link with other polymers or with a mucous membrane, and preferably wherein the mucoadhesive is polyethylene oxide.

40. The modified release solid oral dosage form according to Claim 39, wherein the amount of mucoadhesive in the dosage form is from about 20 to about 80 wt%, about 30 to about 70 wt%, and more preferably about 40 to about 60 wt%, and even more preferably about 50 to about 60 wt%.

41. The modified release solid oral dosage form according to any of Claims 39 or 40, wherein ratio of the memantine or pharmaceutically acceptable salt thereof to mucoadhesive in the dosage form is from about 1 : 1 to about I : 10, preferably from about 1 :2 to about 1 :7, and more preferably from about 1 :2 to about 1 :5.

42. A modified release solid oral dosage form or pharmaceutical formulation according to any of Claims 1-41 for use in the treatment of mild, moderate or severe Alzheimer's dementia, or neuropathic pain.

43. The modified release solid oral dosage form or pharmaceutical formulation according to any of Claims 1-42, wherein the dosage form has a dissolution profile of: not more than 45% at 24 hours, not more than 70% at 48 hours, and not more than 80% at 55 hours; or wherein the dosage form has a dissolution rate of not more than 35% at 24 hours, not more than 70% at 48 hours, or not more than 80% at 55 hours; or, wherein the dosage form has a dissolution rate of not more than 70% at 50 hours, or a dissolution rate of more than 70% at 72 hours or a dissolution rate of more than about 80% at about 96 hours.

44. The modified release solid oral dosage form, or pharmaceutical formulation according to any of Claims I-43, wherein the dosage form comprise the following *in vivo* plasma profile concentrations at steady state: a Cₘₐₓ from about 100 ng/ml to about 145 ng/ml, about 100 ng/ml to about 140 ng/ml, about 100 ng/ml to about 135 ng/ml, about 100 ng/ml to about 130 ng/ml, about 100 ng/ml to about 125 ng/ml, about 100 ng/ml to about 170 ng/ml, preferably from about 100 ng/ml to about 140 ng/ml or from about 100 ng/ml to about 130 ng/ml; a Cₘᵢₙ from about 20 ng/ml to about 125 ng/ml, preferably from about 30 ng/ml to about 125 ng/ml or from about 40 ng/ml to about 125 ng/ml or from about 50 ng/ml to about 125 ng/ml; and an AUCₜₐᵤ from about 10,000 ng h/ml to about 25,000 ng h/ml, preferably from about 14,000 ng h/ml to about 25,000 ng h/ml or from about 15,000 ng h/ml to about 25,000 ng h/ml or from about 16,000 ng h/ml to about 25,000 ng h/ml or from about 17,000 ng h/ml to about 25,000 ng h/ml.

45. The modified release solid oral dosage form, pharmaceutical formulation or method according to any of Claims 1-44, wherein the amount of memantine or a pharmaceutically acceptable salt thereof in the dosage form is up to 200 mg, at least 112 mg, at least 140 mg, at least 160 mg, at least 170 mg, at least 180 mg, at least about 190 mg, from about 112 mg to about 200 mg, from about 140 mg to about 200 mg, or from about 160, 170, 180 or 190 mg to about 200 mg.

46. The modified release solid oral dosage form, pharmaceutical formulation or method according to any of Claims 1-45, wherein the pharmaceutically acceptable salt of memantine is hydrochloride salt or sulfate salt; preferably wherein the memantine is in the form of its hydrochloride salt.

## Patentansprüche

1. Feste orale Darreichungsform mit modifizierter Freisetzung, umfassend eine therapeutisch wirksame Menge von Memantin oder einem pharmazeutisch verträglichen Salz davon und wenigstens einen pharmazeutisch verträglichen ratenkontrollierenden Hilfsstoff, wobei die feste orale Darreichungsform für die Verabreichung mit einem Intervall zwischen Dosen von 5 Tagen oder mehr als 5 Tagen an einen Patienten mit Bedarf daran ausgelegt ist, wobei die feste orale Darreichungsform:
(c) ein *in-vivo*-Plasmaprofil im stationären Zustand bereitstellt, das eine Cₘₐₓ von etwa 160 ng/ml oder weniger, eine Cₘᵢₙ von mehr als etwa 30 ng/ml und eine AUCₜₐᵤ von mehr als etwa 14.000 ng h/ml aufweist, und/oder
(d) ein Auflösungsprofil von: nicht mehr als 45 % nach 24 Stunden, nicht mehr als 70 % nach 48 Stunden und nicht mehr als 80 % nach 55 Stunden aufweist, wie gemessen unter Verwendung eines USP-Typ-1-Auslösungssystems (Korb) bei 100 min⁻¹, 900 ml, 0,2 % NaCl in 0,1 N HCl, pH-Wert = 1,2, bei einer Temperatur von 37 ± 0,5 °C.

2. Feste orale Darreichungsform mit modifizierter Freisetzung gemäß Anspruch 1 oder Anspruch 2, wobei die Darreichungsform Memantin oder ein pharmazeutisch verträgliches Salz davon in einer Menge von wenigstens etwa 112 mg, wenigstens etwa 140 mg, wenigstens etwa 160 mg, wenigstens etwa 170 mg oder wenigstens etwa 190 mg umfasst.

3. Feste orale Darreichungsform mit modifizierter Freisetzung gemäß Anspruch 1 oder Anspruch 2, wobei die Darreichungsform Memantin oder ein pharmazeutisch verträgliches Salz davon in einer Menge von etwa 140 bis etwa 190 mg, etwa 160 bis etwa 190 mg, etwa 170 mg bis etwa 190 mg oder etwa 140 bis etwa 200 mg, etwa 160 bis etwa 200 mg, etwa 170 bis etwa 200 mg oder etwa 190 bis etwa 200 mg umfasst.

4. Feste orale Darreichungsform mit modifizierter Freisetzung, umfassend wenigstens etwa 112 mg oder wenigstens etwa 140 mg an Memantin oder einem pharmazeutisch verträglichen Salz von Memantin und wenigstens einen pharmazeutisch verträglichen ratenkontrollierenden Hilfsstoff, wobei die feste orale Darreichungsform:
(a) ein *in-vivo*-Plasmaprofil im stationären Zustand bereitstellt, das eine Cₘₐₓ von etwa 160 ng/ml oder weniger aufweist, und/oder
(b) ein Auflösungsprofil von: nicht mehr als 45 % nach 24 Stunden, nicht mehr als 70 % nach 48 Stunden und nicht mehr als 80 % nach 55 Stunden aufweist.

5. Feste orale Darreichungsform mit modifizierter Freisetzung gemäß einem der Ansprüche 1-4, ausgelegt für die einmalwöchentliche Verabreichung an einen Patienten mit Bedarf daran.

6. Feste orale Darreichungsform mit modifizierter Freisetzung gemäß Anspruch 5, wobei die wobei die feste orale Darreichungsform:
(a) ein *in-vivo*-Plasmaprofil im stationären Zustand bereitstellt, das eine Cₘₐₓ von etwa 160 ng/ml oder weniger, eine Cₘᵢₙ von mehr als etwa 30 ng/ml, eine Tₘₐₓ von wenigstens etwa 36 und eine AUCₜₐᵤ von mehr als etwa 14.000 ng h/ml aufweist, und/oder
(b) ein Auflösungsprofil von: nicht mehr als 45 % nach 24 Stunden, nicht mehr als 70 % nach 48 Stunden und nicht mehr als 80 % nach 55 Stunden aufweist.

7. Feste orale Darreichungsform mit modifizierter Freisetzung gemäß einem der Ansprüche 4, 5 oder 6, wobei die Darreichungsform Memantin oder ein pharmazeutisch verträgliches Salz davon in einer Menge von wenigstens etwa 160 mg, wenigstens etwa 170 mg oder wenigstens etwa 190 mg umfasst.

8. Feste orale Darreichungsform mit modifizierter Freisetzung gemäß einem der Ansprüche 4, 5, 6 oder 7, wobei die Darreichungsform Memantin oder ein pharmazeutisch verträgliches Salz davon in einer Menge von etwa 140 bis etwa 190 mg, etwa 160 bis etwa 190 mg, etwa 170 mg bis etwa 190 mg oder etwa 140 bis etwa 200 mg, etwa 160 bis etwa 200 mg, etwa 170 bis etwa 200 mg oder etwa 190 bis etwa 200 mg umfasst.

9. Feste orale Darreichungsform mit modifizierter Freisetzung gemäß einem der vorstehenden Ansprüche, wobei die Darreichungsform wenigstens etwa 160 mg an Memantin oder einem pharmazeutisch verträglichen Salz von Memantin umfasst; oder wobei die Darreichungsform wenigstens etwa 170 mg an Memantin oder einem pharmazeutisch verträglichen Salz von Memantin umfasst; oder wobei die Darreichungsform wenigstens etwa 190 mg an Memantin oder einem pharmazeutisch verträglichen Salz von Memantin umfasst; oder wobei die Darreichungsform wenigstens etwa 200 mg an Memantin oder einem pharmazeutisch verträglichen Salz von Memantin umfasst.

10. Feste orale Darreichungsform mit modifizierter Freisetzung gemäß einem der vorstehenden Ansprüche, wobei die feste orale Darreichungsform ein *in-vivo*-Plasmaprofil im stationären Zustand bereitstellt, das eine Cₘₐₓ von etwa 100 ng/ml bis etwa 145 ng/ml, etwa 100 ng/ml bis etwa 135 ng/ml oder etwa 100 ng/ml bis etwa 125 ng/ml aufweist.

11. Feste orale Darreichungsform mit modifizierter Freisetzung gemäß einem der Ansprüche 1-9, wobei die feste orale Darreichungsform ein *in-vivo-*Plasmaprofil im stationären Zustand bereitstellt, das eine Cₘᵢₙ von etwa 30 ng/ml bis etwa 125 ng/ml, etwa 40 ng/ml bis etwa 125 ng/ml oder etwa 50 ng/ml bis etwa 125 ng/ml aufweist.

12. Feste orale Darreichungsform mit modifizierter Freisetzung gemäß einem der Ansprüche 1-9, wobei die feste orale Darreichungsform ein *in-vivo-*Plasmaprofil im stationären Zustand bereitstellt, das eine Cₘᵢₙ von mehr als etwa 30 ng/ml aufweist; oder wobei die feste orale Darreichungsform ein *in-vivo*-Plasmaprofil im stationären Zustand bereitstellt, das eine Cₘᵢₙ von mehr als etwa 40 ng/ml aufweist; wobei die feste orale Darreichungsform ein *in-vivo*-Plasmaprofil im stationären Zustand bereitstellt, das eine Cₘᵢₙ von mehr als etwa 50 ng/ml aufweist.

13. Feste orale Darreichungsform mit modifizierter Freisetzung gemäß einem der vorstehenden Ansprüche, wobei die feste orale Darreichungsform ein *in-vivo*-Plasmaprofil im stationären Zustand bereitstellt, das eine AUCₜₐᵤ von mehr als etwa 14.000 ng h/ml aufweist; oder wobei die feste orale Darreichungsform ein *in-vivo*-Plasmaprofil im stationären Zustand bereitstellt, das eine AUCₜₐᵤ von 15.000 ng h/ml oder mehr und vorzugsweise mehr als etwa 15.000 ng h/ml aufweist; oder wobei die feste orale Darreichungsform ein *in-vivo-*Plasmaprofil im stationären Zustand bereitstellt, das eine AUCₜₐᵤ von mehr als etwa 16.000 ng h/ml aufweist.

14. Feste orale Darreichungsform mit modifizierter Freisetzung gemäß Anspruch 13, wobei die feste orale Darreichungsform ein *in-vivo*-Plasmaprofil im stationären Zustand bereitstellt, das eine AUCₜₐᵤ von etwa 14.000 ng h/ml bis etwa 25.000 ng h/ml aufweist; oder wobei die feste orale Darreichungsform ein *in-vivo*-Plasmaprofil im stationären Zustand bereitstellt, das eine AUCₜₐᵤ von etwa 15.000 ng h/ml bis etwa 25.000 ng h/ml oder von etwa 16.000 ng h/ml bis etwa 25.000 ng h/ml aufweist.

15. Feste orale Darreichungsform mit modifizierter Freisetzung gemäß einem der vorstehenden Ansprüche, wobei die feste orale Darreichungsform eine Auflösungsrate von nicht mehr als 70 % nach 48 Stunden bereitstellt, vorzugsweise nicht mehr als 70 % nach 72 Stunden, und bevorzugter wobei mehr als etwa 80 % nach etwa 96 Stunden erzielt sind; oder wobei die feste orale Darreichungsform ein Auflösungsprofil von nicht mehr als 70 % nach 50 Stunden, nicht mehr als 75 % nach 60 Stunden und mehr als 80 % nach etwa 96 Stunden bereitstellt.

16. Feste orale Darreichungsform mit modifizierter Freisetzung gemäß einem der vorstehenden Ansprüche, wobei das *in-vivo*-Plasmaprofil im stationären Zustand ferner **gekennzeichnet ist durch** eine Memantin-Tₘₐₓ von wenigstens etwa 36 Stunden und vorzugsweise eine Memantin-Tₘₐₓ von etwa 36 bis 96 Stunden; oder eine Tₘₐₓ von etwa 48 bis 72 Stunden.

17. Feste orale Darreichungsform mit modifizierter Freisetzung gemäß einem der vorstehenden Ansprüche, wobei die Darreichungsform für die Verabreichung mit einem Intervall zwischen Dosen von 5 Tagen oder mehr als 5 Tagen, vorzugsweise 6 Tagen an einen Patienten mit Bedarf daran ausgelegt ist, oder für die Verabreichung einmal alle 7 Tage an einen Patienten mit Bedarf daran.

18. Feste orale Darreichungsform mit modifizierter Freisetzung gemäß einem der Ansprüche 1-17, wobei das pharmazeutisch verträgliche Salz von Memantin Hydrochloridsalz oder Sulfatsalz ist und vorzugsweise wobei Memantin in der Form des Hydrochloridsalzes vorliegt.

19. Feste orale Darreichungsform mit modifizierter Freisetzung gemäß einem der Ansprüche 1-18, wobei die Darreichungsform eine Einheitsdarreichungsform ist.

20. Feste orale Darreichungsform mit modifizierter Freisetzung gemäß Anspruch 19, wobei das Memantin oder das pharmazeutisch verträgliche Salz davon sowohl in einer Form für sofortige Freisetzung als auch in einer Form mit verlängerter Freisetzung vorhanden ist.

21. Feste orale Darreichungsform mit modifizierter Freisetzung gemäß Anspruch 20, wobei die Darreichungsform umfasst:
(i) eine Komponente mit sofortiger Freisetzung, umfassend Memantin oder ein pharmazeutisch verträgliches Salz davon mit sofortiger Freisetzung, und
(ii) eine Komponente mit verlängerter Freisetzung, umfassend Memantin oder ein pharmazeutisch verträgliches Salz davon mit verlängerter Freisetzung.

22. Feste orale Darreichungsform mit modifizierter Freisetzung gemäß Anspruch 20 oder Anspruch 21, wobei: die Komponente mit sofortiger Freisetzung (i) in der Form von Kügelchen vorliegt, die Memantin oder ein pharmazeutisch verträgliches Salz davon mit sofortiger Freisetzung umfassen, und die Komponente mit verlängerter Freisetzung (ii) in der Form von Kügelchen vorliegt, die Memantin oder ein pharmazeutisch verträgliches Salz davon mit verlängerter Freisetzung umfassen, wobei die Kügelchen eine Beschichtung für verlängerte Freisetzung umfassen, die wenigstens einen ratenkontrollierenden Hilfsstoff umfasst.

23. Feste orale Darreichungsform mit modifizierter Freisetzung gemäß Anspruch 21 oder Anspruch 22, wobei (i) einen inerten Kern umfasst, vorzugsweise ein Zuckerkügelchen, der mit Memantin oder einem pharmazeutisch verträglichen Salz davon beschichtet ist, und gegebenenfalls ein Bindemittel.

24. Feste orale Darreichungsform mit modifizierter Freisetzung gemäß Anspruch 23, wobei das Bindemittel ausgewählt ist aus der Gruppe bestehend aus: Cellulosepolymeren, Hydroxypropylmethylcellulose, Hydroxypropylcellulose, Methylcellulose, Hydroxyethylcellulose, Polyvinylpyrrolidon, Polyvinylalkohol und Gemischen davon, wobei das Bindemittel vorzugsweise Hydroxypropylmethylcellulose ist.

25. Feste orale Darreichungsform mit modifizierter Freisetzung gemäß einem der Ansprüche 22 bis 24, wobei die Komponente mit verlängerter Freisetzung (ii) durch Beschichten der Kügelchen mit sofortiger Freisetzung (i) gemäß einem der Ansprüche 40 oder 41 mit wenigstens einem ratenkontrollierenden Hilfsstoff hergestellt sind.

26. Feste orale Darreichungsform mit modifizierter Freisetzung gemäß einem der Ansprüche 1-25 wobei wenigstens 90 Gew.-% des Memantins oder eines pharmazeutisch verträglichen Salzes davon in einer Form mit verlängerter Freisetzung vorliegen; vorzugsweise wobei wenigstens 95 Gew.-% des Memantins oder eines pharmazeutisch verträglichen Salzes davon in einer Form mit verlängerter Freisetzung vorliegen.

27. Feste orale Darreichungsform mit modifizierter Freisetzung gemäß einem der Ansprüche 1-26 wobei der ratenkontrollierende Hilfsstoff ein Polymermaterial ist; wobei das Polymermaterial vorzugsweise ausgewählt ist aus Polyethylenoxid, Ethylcellulose (beispielsweise vorzugsweise Ethylcellulose mit einer Viskosität von etwa 4 bis etwa 10 cPs, insbesondere etwa 7 cPs), Hydroxypropylmethylcellulose (HPMC, vorzugsweise mit einer Viskosität von etwa 4 bis etwa 9 cPs, bevorzugter etwa 5 bis etwa 8 cPs und höchst bevorzugt etwa 5 bis etwa 7 cPs und insbesondere etwa 6 cPs), Polyvinylalkohol (PVA, vorzugsweise Polyvinylalkohol 205, 523, 540, 203S, 205S, 523S und 540S), Polyvinylpyrrolidon (PVP, vorzugsweise Povidon K 12, Povidon K 17, Povidon K 25, Povidon K 30 und Povidon K 90, bevorzugter Povidon K 25, Povidon K 30 und Povidon K 90), Polyacrylaten, Polymethacrylaten, Ethylacrylat-Methylmethacrylat-Copolymeren (vorzugsweise Eudragit RS oder NE), Hydroxypropylcellulose (HPC, vorzugsweise mit einer Viskosität von etwa 4 bis etwa 9 cPs, bevorzugter etwa 5 bis etwa 8 cPs und höchst bevorzugt etwa 5 bis etwa 7 cPs) und einem Gemisch davon.

28. Feste orale Darreichungsform mit modifizierter Freisetzung gemäß Anspruch 27, wobei der ratenkontrollierende Hilfsstoff eine Kombination von zwei oder mehr Polymermaterialien ist, vorzugsweise wobei der ratenkontrollierende Hilfsstoff eine Kombination von Ethylcellulose (vorzugsweise mit einer Viskosität von etwa 5 bis etwa 9 cPs, bevorzugter etwa 6 bis etwa 8 cPs) und Hydroxylpropylmethylcellulose (vorzugsweise mit einer Viskosität von etwa 4 bis etwa 9 cPs, bevorzugter etwa 5 bis etwa 8 cPs und höchst bevorzugt etwa 5 bis etwa 7 cPs) ist.

29. Feste orale Darreichungsform mit modifizierter Freisetzung gemäß einem der Ansprüche 27 oder 28, ferner umfassend einen Weichmacher, vorzugsweise wobei der Weichmacher ausgewählt ist aus der Gruppe bestehend aus: Polyethylenglycol, Triethylcitrat, Tributylcitrat, Glycerin, Dibutylsebacat, Triacetin, Diethylphthalat und Gemischen davon, vorzugsweise wobei der Weichmacher Triethylcitrat ist.

30. Feste orale Darreichungsform mit modifizierter Freisetzung gemäß einem der Ansprüche 1-27, wobei der ratenkontrollierende Hilfsstoff ein Ethylacrylat-Methylmethacrylat-Copolymer ist (vorzugsweise Eudragit, bevorzugter Eudragit NE30D, das eine 30%-ige wässrige Dispersion eines Copolymers von Ethylacrylat und Methylmethacrylat ist).

31. Feste orale Darreichungsform mit modifizierter Freisetzung gemäß einem der Ansprüche 1-30, wobei die Gesamtmenge der ratenmodifizierenden Hilfsstoffe bezogen auf das Gesamtgewicht der Darreichungsform von etwa 8 % bis etwa 60 %; von etwa 8 % bis etwa 50 %; von etwa 8 % bis etwa 40 %; von etwa 8 % bis etwa 30 %; von etwa 8 % bis etwa 20 %; von etwa 50 % bis etwa 60 %>; von etwa 19 %> bis etwa 40 %>; von etwa 19 %> bis etwa 30 %>; von etwa 19% bis etwa 25%; von etwa 30 %> bis etwa 60 %>; von etwa 30 %> bis etwa 50 %>; von etwa 30 % bis etwa 40 %>; von etwa 40 %> bis etwa 60 %>; oder von etwa 50 %> bis etwa 60 % beträgt.

32. Feste orale Darreichungsform mit modifizierter Freisetzung gemäß einem der Ansprüche 21-31, wobei die Menge an ratenmodifizierenden Hilfsstoff etwa 10 bis etwa 50 Gew.-%, vorzugsweise etwa 10 bis etwa 45 Gew.-% und bevorzugter etwa 15 bis etwa 40 Gew.-% der Komponente mit verlängerter Freisetzung der Darreichungsform beträgt.

33. Feste orale Darreichungsform mit modifizierter Freisetzung gemäß einem der Ansprüche 21-32, wobei die Menge an Memantin oder einem pharmazeutisch verträglichen Salz von Memantin in der Komponente mit verlängerter Freisetzung etwa 10 bis etwa 60 Gew.-%>, vorzugsweise etwa 10 bis etwa 55 Gew.-% und bevorzugter etwa 10 bis etwa 50 Gew.-% bezogen auf das Gewicht der Komponente mit verlängerter Freisetzung beträgt.

34. Feste orale Darreichungsform mit modifizierter Freisetzung gemäß einem der Ansprüche 21-33, wobei in der Komponente mit verlängerter Freisetzung das Gewichtsverhältnis von ratenmodifizierenden Hilfsstoff(en) zu Memantin oder dem pharmazeutisch verträglichen Salz davon von etwa 1:0,2 bis etwa 1:5,0 beträgt, vorzugsweise von etwa 1:0,3 bis etwa 1:3,0 und bevorzugter etwa 1:0,3 bis etwa 1:2,8.

35. Feste orale Darreichungsform mit modifizierter Freisetzung gemäß einem der Ansprüche 29-34, wobei in der Komponente mit verlängerter Freisetzung das Gewichtsverhältnis von Weichmacher zu ratenmodifizierenden Hilfsstoff(en) von etwa 1:2 bis etwa 1:10 beträgt, vorzugsweise von etwa 1:3 bis etwa 1:8 und bevorzugter etwa 1:4 bis etwa 1:7.

36. Feste orale Darreichungsform mit modifizierter Freisetzung gemäß einem der Ansprüche 1-35, ferner umfassend ein mukoadhäsives Mittel; vorzugsweise wobei das mukosadhäsive Mittel ausgewählt ist aus der Gruppe bestehend aus wasserlöslichen oder wasserunlöslichen hydrophilen Polymeren, Polymeren, die quellbare Netzwerke aufweisen, Hydrogelen, und Polymeren mit Gruppen, die mit anderen Polymeren oder mit einer Schleimhautmembran vernetzen können, vorzugsweise wobei das mukosadhäsive Mittel Polyethylenoxid ist.

37. Feste orale Darreichungsform mit modifizierter Freisetzung gemäß Anspruch 36, wobei das mukoadhäsive Mittel in einer Menge von etwa 5 bis etwa 60 Gew.-%, etwa 5 bis etwa 50 Gew.-%, etwa 5 bis etwa 40 Gew.-%, etwa 5 bis etwa 20 Gew.-%, etwa 5 bis etwa 15 Gew.-% und höchst bevorzugt etwa 5 bis etwa 10 Gew.-% des Gesamtgewichts der Darreichungsform vorhanden ist.

38. Feste orale Darreichungsform mit modifizierter Freisetzung gemäß einem der Ansprüche 36 bis 37, wobei das Gewichtsverhältnis von Memantin oder dem pharmazeutisch verträglichen Salz davon zu dem mukoadhäsiven Mittel von etwa 1:2 bis etwa 1:4 beträgt, vorzugsweise etwa 1:4.

39. Feste orale Darreichungsform mit modifizierter Freisetzung gemäß einem der Ansprüche 1-19, wobei das Memantin oder das pharmazeutisch verträgliche Salz davon in einer Matrix bereitgestellt ist, die ein mukoadhäsives Mittel enthält; vorzugsweise wobei das mukoadhäsive Mittel ausgewählt ist aus der Gruppe bestehend aus wasserlöslichen oder wasserunlöslichen hydrophilen Polymeren, Polymeren, die quellbare Netzwerke aufweisen, Hydrogelen, und Polymeren mit Gruppen, die mit anderen Polymeren oder mit einer Schleimhautmembran vernetzen können, vorzugsweise wobei das mukosadhäsive Mittel Polyethylenoxid ist.

40. Feste orale Darreichungsform mit modifizierter Freisetzung gemäß Anspruch 39, wobei die Menge an mukoadhäsivem Mittel in der Darreichungsform von etwa 20 bis etwa 80 Gew.-% beträgt, etwa 30 bis etwa 70 Gew.-% und bevorzugter etwa 40 bis etwa 60 Gew.-% und noch bevorzugter etwa 50 bis etwa 60 Gew.-%.

41. Feste orale Darreichungsform mit modifizierter Freisetzung gemäß einem der Ansprüche 39 oder 40, wobei das Verhältnis von Memantin oder dem pharmazeutisch verträglichen Salz davon zu dem mukoadhäsiven Mittel in der Darreichungsform von etwa 1:1 bis etwa 1:10 beträgt, vorzugsweise von etwa 1:2 bis etwa 1:7 und bevorzugter von etwa 1:2 bis etwa 1:5.

42. Feste orale Darreichungsform mit modifizierter Freisetzung gemäß einem der Ansprüche 1-41 für die Verwendung bei der Behandlung von milder, mäßiger oder schwerer Alzheimer-Demenz oder neuropathischem Schmerz.

43. Feste orale Darreichungsform mit modifizierter Freisetzung oder pharmazeutische Formulierung gemäß einem der Ansprüche 1-42, wobei die Darreichungsform ein Auflösungsprofil aufweist von: nicht mehr als 45 % nach 24 Stunden, nicht mehr als 70 % nach 48 Stunden und nicht mehr als 80 % nach 55 Stunden; oder wobei die Darreichungsform eine Auflösungsrate von nicht mehr als 35 % nach 24 Stunden, nicht mehr als 70 % nach 48 Stunden und nicht mehr als 80 % nach 55 Stunden aufweist; oder wobei die Darreichungsform eine Auflösungsrate von nicht mehr als 70 % nach 50 Stunden oder eine Auflösungsrate von mehr als 70 % nach 72 Stunden oder eine Auflösungsrate von mehr als etwa 80 % nach etwa 96 Stunden aufweist.

44. Feste orale Darreichungsform mit modifizierter Freisetzung oder pharmazeutische Formulierung gemäß einem der Ansprüche 1-43, wobei die Darreichungsform folgende *in-vivo-*Plasmaprofilkonzentrationen im stationären Zustand aufweist: eine Cₘₐₓ von etwa 100 ng/ml bis etwa 145 ng/ml, etwa 100 ng/ml bis etwa 140 ng/ml, etwa 100 ng/ml bis etwa 135 ng/ml, etwa 100 ng/ml bis etwa 130 ng/ml, etwa 100 ng/ml bis etwa 125 ng/ml, etwa 100 ng/ml bis etwa 170 ng/ml, vorzugsweise von etwa 100 ng/ml bis etwa 140 ng/ml oder von etwa 100 ng/ml bis etwa 130 ng/ml; eine Cₘᵢₙ von etwa 20 ng/ml bis etwa 125 ng/ml, vorzugsweise von etwa 30 ng/ml bis etwa 125 ng/ml oder von etwa 40 ng/ml bis etwa 125 ng/ml oder von etwa 50 ng/ml bis etwa 125 ng/ml; und eine AUCₜₐᵤ von etwa 10.000 ng h/ml bis etwa 25,000 ng h/ml, vorzugsweise von etwa 14.000 ng h/ml bis etwa 25.000 ng h/ml oder von etwa 15.000 ng h/ml bis etwa 25.000 ng h/ml oder von etwa 16.000 ng h/ml bis etwa 25.000 ng h/ml oder von etwa 17.000 ng h/ml bis etwa 25.000 ng h/ml.

45. Feste orale Darreichungsform mit modifizierter Freisetzung oder pharmazeutische Formulierung gemäß einem der Ansprüche 1-44, wobei die Menge an Memantin oder einem pharmazeutisch verträglichen Salz davon in der Darreichungsform bis zu 200 mg beträgt, wenigstens 112 mg, wenigstens 140 mg, wenigstens 160 mg, wenigstens 170 mg, wenigstens 180 mg, wenigstens etwa 190 mg, von etwa 112 mg bis etwa 200 mg, von etwa 140 mg bis etwa 200 mg oder von etwa 160, 170, 180 oder 190 mg bis etwa 200 mg.

46. Feste orale Darreichungsform mit modifizierter Freisetzung oder pharmazeutische Formulierung gemäß einem der Ansprüche 1-45, wobei das pharmazeutisch verträgliche Salz von Memantin Hydrochloridsalz oder Sulfatsalz ist; vorzugsweise wobei das Memantin in der Form seines Hydrochloridsalzes vorliegt.

## Revendications

1. Forme posologique orale solide à libération modifiée comprenant une quantité à effet thérapeutique de mémantine ou un sel pharmaceutiquement acceptable de celle-ci, et au moins un excipient de contrôle de la vitesse pharmaceutiquement acceptable, la forme posologique orale solide étant adaptée à une administration à un patient qui en a besoin avec un intervalle entre les doses de 5 jours ou supérieur à 5 jours, et la forme posologique orale solide :
(c) fournissant un profil plasmatique *in vivo* à l'état d'équilibre présentant une valeur Cₘₐₓ d'environ 160 ng/ml ou inférieure, une valeur Cₘᵢₙ supérieure à environ 30 ng/ml et une valeur AUCₜₐᵤ supérieure à environ 14 000 ng.h/ml, et/ou
(d) ayant un profil de dissolution d'au plus 45 % après 24 h, d'au plus 70 % après 48 h et d'au plus 80 % après 55 h, mesuré à l'aide d'un système de dissolution USP de type 1 (panier) avec les paramètres suivants : 100 tr/min, 900 ml, NaCl à 0,2 % dans HCl à 0,1 N, pH = 1,2, à une température de 37 ± 0,5 °C.

2. Forme posologique orale solide à libération modifiée selon les revendications 1 ou 2, la forme posologique comprenant de la mémantine ou un sel pharmaceutiquement acceptable de celle-ci en une quantité d'au moins environ 112 mg, d'au moins environ 140 mg, d'au moins environ 160 mg, d'au moins environ 170 mg ou d'au moins environ 190 mg.

3. Forme posologique orale solide à libération modifiée selon les revendications 1 ou 2, la forme posologique comprenant de la mémantine ou un sel pharmaceutiquement acceptable de celle-ci en une quantité comprise entre environ 140 et environ 190 mg, entre environ 160 et environ 190 mg, entre environ 170 mg et environ 190 mg ou entre environ 140 et environ 200 mg, entre environ 160 et environ 200 mg, entre environ 170 et environ 200 mg ou entre environ 190 et environ 200 mg.

4. Forme posologique orale solide à libération modifiée comprenant au moins environ 112 mg ou au moins environ 140 mg de mémantine ou d'un sel pharmaceutiquement acceptable de mémantine, et au moins un excipient de contrôle de la vitesse pharmaceutiquement acceptable, la forme posologique orale solide :
(a) fournissant un profil plasmatique *in vivo* à l'état d'équilibre présentant une valeur Cₘₐₓ d'environ 160 ng/ml ou inférieure, et/ou
(b) ayant un profil de dissolution d'au plus 45 % après 24 h, d'au plus 70 % après 48 h et d'au plus 80 % après 55 h.

5. Forme posologique orale solide à libération modifiée selon l'une quelconque des revendications 1 à 4 adaptée à une administration à un patient qui en a besoin une fois par semaine.

6. Forme posologique orale solide à libération modifiée selon la revendication 5, la forme posologique orale solide :
(a) fournissant un profil plasmatique *in vivo* à l'état d'équilibre présentant une valeur Cₘₐₓ d'environ 160 ng/ml ou inférieure, une valeur Cₘᵢₙ supérieure à environ 30 ng/ml, une valeur Tₘₐₓ d'au moins environ 36 et une valeur AUCₜₐᵤ supérieure à environ 14 000 ng.h/ml, et/ou
(b) ayant un profil de dissolution d'au plus 45 % après 24 h, d'au plus 70 % après 48 h et d'au plus 80 % après 55 h.

7. Forme posologique orale solide à libération modifiée selon l'une quelconque des revendications 4, 5 ou 6, la forme posologique comprenant de la mémantine ou un sel pharmaceutiquement acceptable de celle-ci en une quantité d'au moins environ 160 mg, d'au moins environ 170 mg ou d'au moins environ 190 mg.

8. Forme posologique orale solide à libération modifiée selon l'une quelconque des revendications 4, 5, 6 ou 7, la forme posologique comprenant de la mémantine ou un sel pharmaceutiquement acceptable de celle-ci en une quantité comprise entre environ 140 et environ 190 mg, entre environ 160 et environ 190 mg, entre environ 170 mg et environ 190 mg ou entre environ 140 et environ 200 mg, entre environ 160 et environ 200 mg, entre environ 170 et environ 200 mg ou entre environ 190 et environ 200 mg.

9. Forme posologique orale solide à libération modifiée selon l'une quelconque des revendications précédentes, la forme posologique comprenant au moins environ 160 mg de mémantine ou d'un sel pharmaceutiquement acceptable de mémantine ; ou la forme posologique comprenant au moins environ 170 mg de mémantine ou d'un sel pharmaceutiquement acceptable de celle-ci ; ou la forme posologique comprenant au moins environ 190 mg de mémantine ou d'un sel pharmaceutiquement acceptable de mémantine ; ou la forme posologique comprenant jusqu'à environ 200 mg de mémantine ou d'un sel pharmaceutiquement acceptable de mémantine.

10. Forme posologique orale solide à libération modifiée selon l'une quelconque des revendications précédentes, la forme posologique fournissant un profil plasmatique *in vivo* à l'état d'équilibre présentant une valeur Cₘₐₓ comprise entre environ 100 ng/ml et environ 145 ng/ml, entre environ 100 ng/ml et environ 135 ng/ml ou entre environ 100 ng/ml et environ 125 ng/ml.

11. Forme posologique orale solide à libération modifiée selon l'une quelconque des revendications 1 à 9, la forme posologique fournissant un profil plasmatique *in vivo* à l'état d'équilibre présentant une valeur Cₘᵢₙ comprise entre environ 30 ng/ml et environ 125 ng/ml, entre environ 40 ng/ml et environ 125 ng/ml ou entre environ 50 ng/ml et environ 125 ng/ml.

12. Forme posologique orale solide à libération modifiée selon l'une quelconque des revendications 1 à 9, la forme posologique fournissant un profil plasmatique *in vivo* à l'état d'équilibre présentant une valeur Cₘᵢₙ supérieure à environ 30 ng/ml ; ou la forme posologique fournissant un profil plasmatique *in vivo* à l'état d'équilibre présentant une valeur Cₘᵢₙ supérieure à environ 40 ng/ml ; ou la forme posologique fournissant un profil plasmatique *in vivo* à l'état d'équilibre présentant une valeur Cₘᵢₙ supérieure à environ 50 ng/ml.

13. Forme posologique orale solide à libération modifiée selon l'une quelconque des revendications précédentes, la forme posologique fournissant un profil plasmatique *in vivo* à l'état d'équilibre présentant une valeur AUCₜₐᵤ supérieure à environ 14 000 ng.h/ml ; ou la forme posologique fournissant un profil plasmatique *in vivo* à l'état d'équilibre présentant une valeur AUCₜₐᵤ de 15 000 ng.h/ml ou supérieure et de préférence supérieure à environ 15 000 ng.h/ml ; ou la forme posologique fournissant un profil plasmatique *in vivo* à l'état d'équilibre présentant une valeur AUCₜₐᵤ supérieure à 16 000 ng.h/ml.

14. Forme posologique orale solide à libération modifiée selon la revendication 13, la forme posologique fournissant un profil plasmatique *in vivo* à l'état d'équilibre présentant une valeur AUCₜₐᵤ comprise entre environ 14 000 ng.h/ml et environ 25 000 ng.h/ml ; ou la forme posologique fournissant un profil plasmatique *in vivo* à l'état d'équilibre présentant une valeur AUCₜₐᵤ comprise entre environ 15 000 ng.h/ml et environ 25 000 ng.h/ml ou entre environ 16 000 ng.h/ml et environ 25 000 ng.h/ml.

15. Forme posologique orale solide à libération modifiée selon l'une quelconque des revendications précédentes, la forme posologique fournissant un taux de dissolution d'au plus 70 % après 48 h, de préférence d'au plus 70 % après 72 h et plus préférentiellement supérieur à environ 80 % après environ 96 h ; ou la forme posologique fournissant un profil de dissolution d'au plus 70 % après 50 h, d'au plus 75 % après 60 h et supérieur à environ 80 % après environ 96 h.

16. Forme posologique orale solide à libération modifiée selon l'une quelconque des revendications précédentes, le profil plasmatique *in vivo* à l'état d'équilibre étant **caractérisé en outre par** une valeur Tₘₐₓ de mémantine d'au moins environ 36 h, et de préférence par une valeur Tₘₐₓ de mémantine comprise entre environ 36 et 96 h ou par une valeur Tₘₐₓ comprise entre environ 48 et 72 h.

17. Forme posologique orale solide à libération modifiée selon l'une quelconque des revendications précédentes, la forme posologique étant adaptée à une administration à un patient qui en a besoin avec un intervalle entre les doses de 5 jours ou supérieur à 5 jours, de préférence de 6 jours, ou à une administration à un patient qui en a besoin une fois tous les 7 jours.

18. Forme posologique orale solide à libération modifiée selon l'une quelconque des revendications 1 à 17, le sel pharmaceutiquement acceptable de mémantine étant un sel chlorhydrate ou un sel sulfate et la mémantine étant de préférence sous la forme d'un sel chlorhydrate.

19. Forme posologique orale solide à libération modifiée selon l'une quelconque des revendications 1 à 18, la forme posologique étant une forme posologique unitaire.

20. Forme posologique orale solide à libération modifiée selon la revendication 19, la mémantine ou le sel pharmaceutiquement acceptable de celle-ci étant présent à la fois sous forme à libération immédiate et sous forme à libération prolongée.

21. Forme posologique orale solide à libération modifiée selon la revendication 20, la forme posologique comprenant :
(i) un composant à libération immédiate comprenant de la mémantine à libération immédiate ou un sel pharmaceutiquement acceptable de celle-ci, et
(ii) un composant à libération prolongée comprenant de la mémantine à libération prolongée ou un sel pharmaceutiquement acceptable de celle-ci.

22. Forme posologique orale solide à libération modifiée selon les revendications 20 ou 21, le composant à libération immédiate (i) étant sous forme de billes comprenant de la mémantine à libération immédiate ou un sel pharmaceutiquement acceptable de celle-ci, et le composant à libération prolongée (ii) étant sous forme de billes comprenant de la mémantine à libération prolongée ou un sel pharmaceutiquement acceptable de celle-ci, les billes comprenant un revêtement à libération prolongée contenant au moins un excipient de contrôle de la vitesse.

23. Forme posologique orale solide à libération modifiée selon les revendications 21 ou 22, (i) comprenant un noyau inerte, de préférence une sphère de sucre, enrobée de mémantine ou d'un sel pharmaceutiquement acceptable de celle-ci, et en option un liant.

24. Forme posologique orale solide à libération modifiée selon la revendication 23, le liant étant choisi dans le groupe constitué par les polymères de cellulose, l'hydroxypropylméthylcellulose, l'hydroxypropylcellulose, la méthylcellulose, l'hydroxyéthylcellulose, la polyvinylpyrrolidone, l'alcool polyvinylique et des mélanges de ceux-ci, et le liant étant de préférence l'hydroxypropylméthylcellulose.

25. Forme posologique orale solide à libération modifiée selon l'une quelconque des revendications 22 à 24, le composant à libération prolongée (ii) étant préparé en enrobant les billes à libération immédiate de composant (i) tel que défini dans l'une quelconque des revendications 40 ou 41 avec au moins un excipient de contrôle de la vitesse.

26. Forme posologique orale solide à libération modifiée selon l'une quelconque des revendications 1 à 25, au moins 90 % en poids de la mémantine ou d'un sel pharmaceutiquement acceptable de celle-ci étant sous une forme à libération prolongée ; et au moins 95 % en poids de la mémantine ou d'un sel pharmaceutiquement acceptable de celle-ci étant de préférence sous une forme à libération prolongée.

27. Forme posologique orale solide à libération modifiée selon l'une quelconque des revendications 1 à 26, l'excipient de contrôle de la vitesse étant un matériau polymère ; le matériau polymère étant choisi de préférence parmi l'oxyde de polyéthylène, l'éthylcellulose (par exemple, de préférence de l'éthylcellulose ayant une viscosité comprise entre environ 4 et environ 10 cPs, en particulier d'environ 7 cPs), l'hydroxypropylméthylcellulose (HPMC, ayant de préférence une viscosité comprise entre environ 4 et environ 9 cPs, plus préférentiellement entre environ 5 et environ 8 cPs et de manière préférée entre toutes entre environ 5 et environ 7 cPs et en particulier d'environ 6 cPs), l'alcool polyvinylique (APV, de préférence l'alcool polyvinylique 205, 523, 540, 203S, 205S, 523S et 540S), la polyvinylpyrrolidone (PVP, de préférence Povidone K 12, Povidone K 17, Povidone K 25, Povidone K 30 et Povidone K 90, plus préférentiellement Povidone K 25, Povidone K 30 et Povidone K 90), les polyacrylates, les polyméthacrylates, les copolymères acrylate d'éthyle-méthacrylate de méthyle (de préférence Eudragit RS ou NE), l'hydroxypropylcellulose (HPC, ayant de préférence une viscosité comprise entre environ 4 et environ 9 cPs, plus préférentiellement entre environ 5 et environ 8 cPs et de manière préférée entre toutes entre environ 5 et environ 7 cPs) et un mélange de ceux-ci.

28. Forme posologique orale solide à libération modifiée selon la revendication 27, l'excipient de contrôle de la vitesse étant une combinaison de deux matériaux polymères, l'excipient de contrôle de la vitesse étant de préférence une combinaison d'éthylcellulose (ayant de préférence une viscosité comprise entre environ 5 et environ 9 cPs, plus préférentiellement entre environ 6 et environ 8 cPs) et l'hydroxypropylméthylcellulose (ayant de préférence une viscosité comprise entre environ 4 et environ 9 cPs, plus préférentiellement entre environ 5 et environ 8 cPs et de manière préférée entre toutes entre environ 5 et environ 7 cPs).

29. Forme posologique orale solide à libération modifiée selon l'une quelconque des revendications 27 ou 28, comprenant en outre un plastifiant ; le plastifiant étant de préférence choisi dans le groupe constitué par le polyéthylène glycol, le citrate de triéthyle, le citrate de tributyle, la glycérine, le sébacate de dibutyle, la triacétine, le phtalate de diéthyle et des mélanges de ceux-ci, et le plastifiant étant de préférence le citrate de triéthyle.

30. Forme posologique orale solide à libération modifiée selon l'une quelconque des revendications 1 à 27, l'excipient de contrôle de la vitesse étant un copolymère acrylate d'éthyle-méthacrylate de méthyle (de préférence Eudragit et plus préférentiellement Eudragit NE30D, qui est une dispersion aqueuse à 30 % d'un copolymère d'acrylate d'éthyle et de méthacrylate de méthyle).

31. Forme posologique orale solide à libération modifiée selon l'une quelconque des revendications 1 à 30, la quantité totale d'excipient de contrôle de la vitesses par rapport au poids total de la forme posologique étant comprise entre environ 8 % et environ 60 %; entre environ 8 % et environ 50 %; entre environ 8 % et environ 40 %; entre environ 8 % et environ 30 %; entre environ 8 % et environ 20 %; entre environ 50 % et environ 60 %>; entre environ 19 %> et environ 40 >%; entre environ 19 %> et environ 30 %>; entre environ 19%> et à environ 25 %; entre environ 30 %> et environ 60 %>; entre environ 30 %> et environ 50 %>; entre environ 30 % et environ 40 %>; entre environ 40 %> et environ 60 %>; ou entre environ 50 %> et environ 60 %.

32. Forme posologique orale solide à libération modifiée selon l'une quelconque des revendications 21 à 31, la quantité d'excipient de contrôle de la vitesse étant comprise entre environ 10 et environ 50 % en poids, de préférence entre environ 10 et environ 45 % en poids et plus préférentiellement entre environ 15 et environ 40 % en poids du composant à libération prolongée de la forme posologique.

33. Forme posologique orale solide à libération modifiée selon l'une quelconque des revendications 21 à 32, la quantité de mémantine ou d'un sel pharmaceutiquement acceptable de mémantine dans le composant à libération prolongée étant comprise entre environ 10 et environ 60 %> en poids, de préférence entre environ 10 et environ 55 % en poids et plus préférentiellement entre environ 10 et environ 50 % en poids par rapport au poids du composant à libération prolongée.

34. Forme posologique orale solide à libération modifiée selon l'une quelconque des revendications 21 à 33, le rapport en poids entre le ou les excipients de contrôle de la vitesse et la mémantine ou le sel pharmaceutiquement acceptable de celle-ci dans le composant à libération prolongée étant compris entre environ 1:0,2 et environ 1:5,0, de préférence entre environ 1:0,3 et environ 1:3,0 et plus préférentiellement entre environ 1:0,3 et environ 1:2,8.

35. Forme posologique orale solide à libération modifiée selon l'une quelconque des revendications 29 à 34, le rapport en poids entre le plastifiant et le ou les excipients de contrôle de la vitesse dans le composant à libération prolongée étant compris entre environ 1:2 et environ 1:10, de préférence entre environ 1:3 et environ 1:8 et plus préférentiellement entre environ 1:4 et environ 1:7.

36. Forme posologique orale solide à libération modifiée selon l'une quelconque des revendications 1 à 35, comprenant en outre un mucoadhésif ; le mucoadhésif étant de préférence choisi dans le groupe constitué par des polymères hydrophiles solubles ou insolubles dans l'eau, des polymères qui ont des réseaux gonflables, des hydrogels, et des polymères possédant des groupes qui peuvent se réticuler avec d'autres polymères ou avec une muqueuse, et le mucoadhésif étant de préférence l'oxyde de polyéthylène.

37. Forme posologique orale solide à libération modifiée selon la revendication 36, le mucoadhésif étant présent en une quantité comprise entre environ 5 et environ 60 % en poids, entre environ 5 et environ 50 % en poids, entre environ 5 et environ 40 % en poids, entre environ 5 et environ 20 % en poids, entre environ 5 et environ 15 % en poids et de manière préférée entre toutes entre environ 5 et environ 10 % en poids, du poids total de la forme posologique.

38. Forme posologique orale solide à libération modifiée selon l'une quelconque des revendications 36 ou 37, le rapport en poids entre la mémantine ou le sel pharmaceutiquement acceptable de celle-ci et le mucoadhésif étant compris entre environ 1:2 et environ 1:4, de préférence d'environ 1:4.

39. Forme posologique orale solide à libération modifiée selon l'une quelconque des revendications 1 à 19, la mémantine ou le sel pharmaceutiquement acceptable de celle-ci étant fourni dans une matrice comprenant un agent mucoadhésif ; le mucoadhésif étant choisi de préférence dans le groupe constitué par des polymères hydrophiles solubles ou insolubles dans l'eau, des polymères qui ont des réseaux gonflables, des hydrogels, et des polymères possédant des groupes qui peuvent se réticuler avec d'autres polymères ou avec une muqueuse, et le mucoadhésif étant de préférence l'oxyde de polyéthylène.

40. Forme posologique orale solide à libération modifiée selon la revendication 39, la quantité de mucoadhésif dans la forme posologique étant comprise entre environ 20 et environ 80 % en poids, entre environ 30 et environ 70 % en poids, plus préférentiellement entre environ 40 et environ 60 % en poids, et encore plus préférentiellement entre environ 50 et environ 60 % en poids.

41. Forme posologique orale solide à libération modifiée selon l'une quelconque des revendications 39 ou 40, le rapport entre la mémantine ou le sel pharmaceutiquement acceptable de celle-ci et le mucoadhésif dans la forme posologique étant compris entre environ 1:1 et environ 1:10, de préférence entre environ 1:2 et environ 1:7 et plus préférentiellement entre environ 1:2 et environ 1:5.

42. Forme posologique orale solide à libération modifiée ou formulation pharmaceutique selon l'une quelconque des revendications 1 à 41 destinée à être utilisée dans le traitement de la maladie d'Alzheimer légère, modérée ou sévère ou des douleurs neuropathiques.

43. Forme posologique orale solide à libération modifiée ou formulation pharmaceutique selon l'une quelconque des revendications 1 à 42, la forme posologique ayant un profil de dissolution d'au plus 45 % après 24 h, d'au plus 70 % après 48 h et d'au plus 80 % après 55 h ; ou la forme posologique ayant un taux de dissolution d'au plus 35 % après 24 h, d'au plus 70 % après 48 h ou d'au plus 80 % après 55 h ; ou la forme posologique ayant un taux de dissolution d'au plus 70 % après 50 h ou un taux de dissolution supérieur à 70 % après 72 h ou un taux de dissolution supérieur à environ 80 % après environ 96 h.

44. Forme posologique orale solide à libération modifiée ou formulation pharmaceutique selon l'une quelconque des revendications 1 à 43, la forme posologique comprenant les concentrations plasmatiques *in vivo* à l'état d'équilibre suivantes : une valeur Cₘₐₓ comprise entre environ 100 ng/ml et environ 145 ng/ml, entre environ 100 ng/ml et environ 140 ng/ml, entre environ 100 ng/ml et environ 135 ng/ml, entre environ 100 ng/ml et environ 130 ng/ml, entre environ 100 ng/ml et environ 125 ng/ml, entre environ 100 ng/ml et environ 170 ng/ml, de préférence entre environ 100 ng/ml et environ 140 ng/ml ou entre environ 100 ng/ml et environ 130 ng/ml ; une valeur Cₘᵢₙ comprise entre environ 20 ng/ml et environ 125 ng/ml, de préférence entre environ 30 ng/ml et environ 125 ng/ml ou entre environ 40 ng/ml et environ 125 ng/ml ou entre environ 50 ng/ml et environ 125 ng/ml ; et une valeur AUCₜₐᵤ comprise entre environ 10 000 ng.h/ml et environ 25 000 ng.h/ml, de préférence entre environ 14 000 ng.h/ml et environ 25 000 ng.h/ml ou entre environ 15 000 ng.h/ml et environ 25 000 ng.h/ml ou entre environ 16 000 ng.h/ml et environ 25 000 ng.h/ml ou entre environ 17 000 ng.h/ml et environ 25 000 ng.h/ml.

45. Forme posologique orale solide à libération modifiée ou formulation pharmaceutique selon l'une quelconque des revendications 1 à 44, la quantité de mémantine ou d'un sel pharmaceutiquement acceptable de celle-ci dans la forme posologique étant d'au plus 200 mg, d'au moins 112 mg, d'au moins 140 mg, d'au moins 160 mg, d'au moins 170 mg, d'au moins 180 mg, d'au moins environ 190 mg, comprise entre environ 112 mg et environ 200 mg, entre environ 140 mg et environ 200 mg ou entre environ 160, 170, 180 ou 190 mg et environ 200 mg.

46. Forme posologique orale solide à libération modifiée ou formulation pharmaceutique selon l'une quelconque des revendications 1 à 45, le sel pharmaceutiquement acceptable de mémantine étant un sel chlorhydrate ou un sel sulfate ; la mémantine étant de préférence sous forme de son sel chlorhydrate.
